# EUROPEAN PATENT SPECIFICATION

(11) **EP 0 726 915 B1**
(45) Date of publication and mention of the grant of the patent: **09.10.2002**
(21) Application number: 95900671.9
(22) Date of filing: 04.11.1994
(51) Int. Cl.: C07K 16/28

(54) **A LIGAND CAPABLE OF BINDING TO THE ADENOSINE DEAMINASE BINDING-SITE OF CD26**
LIGAND FÄHIG ZUR BINDUNG AN DER ADENOSINEDEAMINASE-BINDUNGSSTELLE VON CD26
LIGAND APTE A SE LIER AU SITE DE LIAISON D'ADENOSINE DESAMINASE DE CD26

(30) Priority: 04.11.1993 EP 93203093; 28.02.1994 NL 9400309
(43) Date of publication of application: 21.08.1996
(60) Divisional application: 01203730.5
(73) Proprietor: EUROGENETICS N.V., 3980 Tessenderlo (BE); UNIVERSITAIRE INSTELLING ANTWERPEN, B-2610 Wilrijk (BE)
(72) Inventor: DE MEESTER, Ingrid, B-2610 Wilrijk (BE); VAN HAM, Guido, B-2500 Lier (BE); KESTENS, Luc, B-2020 Antwerpen 2 (BE); VANHOOF, Greta, B-2640 Mortsel (BE); VERHELST, Peggy, B-2018 Antwerpen (BE); GOOSSENS, Filip, B-9160 Lokeren (BE); HENDRIKS, Dirk, B-2630 Aartselaar (BE); BORLOO, Marianne, B-2100 Deurne (BE); HAEMERS, Achiel, B-9000 Gent (BE); BOSMANS, Eugène, B-3520 Zonhoven (BE); SCHARPE, Simon, B-9280 Wieze (BE)
(74) Representative: Van Someren, Petronella F. H. M.
(86) International application number: EP9403632
(87) International publication number: WO95012618

(56) References cited:
- TISSUE ANTIGENS, vol.42, no.4, 1993, COPENHAGEN, DENMARK page 242 I. DE MEESTER ET AL. 'Down-regulation of CD26 surface expression by anti-TA5.9 monoclonal antibody.'
- IMMUNOBIOLOGY, vol.188, no.1-2, June 1993, STUTTGART, GERMANY pages 145 - 158 I. DE MEESTER ET AL. 'Antibody binding profile of purified and cell-bound CD26. Designation of BT5/9 and TA5.9 to the CD26 cluster.' cited in the application
- THE JOURNAL OF EXPERIMENTAL MEDICINE, vol.177, no.4, 1 April 1993, NEW YORK NY, USA pages 1135 - 1143 M. MORRISON ET AL. 'A marker for neoplastic progression of human melanocytes is a cell surface ectopeptidase.' cited in the application
- THE SCANDINAVIAN JOURNAL OF IMMUNOLOGY, vol.31, no.4, April 1990, OXFORD, GB pages 429 - 435 A. ULMER ET AL. 'CD26 antigen is a surface dipeptidylpeptidase IV (DPPIV) as characterized by monoclonal antibodies clone TII-19-4-7 and 4EL1C7.' cited in the application
- MOLECULAR IMMUNOLOGY, vol.29, no.2, February 1992, OXFORD, GB pages 183 - 192 Y. TORIMOTO ET AL. 'Biochemical characterization of CD26 (dipeptidyl peptidase IV): functional comparison of distinct epitopes recognized by various anti-CD26 monoclonal antibodies.'
- EUROPEAN JOURNAL OF IMMUNOLOGY, vol.24, no.3, March 1994, WEINHEIM, GERMANY pages 566 - 570 I. DE MEESTER ET AL. 'Binding of adenosine deaminase to the lymphocyte surface via CD26.'

## Description

The invention relates to a monoclonal antibody or functional derivative thereof, capable of binding to the adenosine deaminase binding-site of CD26. Furthermore, the invention concerns the use of the antibody or derivatives in various applications

Apart from the classic T-cell receptor-CD3-complex (TcR-CD3), different other molecules appear to be involved in co-stimulation of the lymphocytes. CD26 is such a plasma membrane molecule. It is an activation marker, which means that its expression is increased on active T-cells.

The enzyme adenosine deaminase (ADA) catalyses the irreversible deamination of adenosine and 2' deoxy-adenosine to inosine and 2'deoxyinosine, respectively. An hereditary shortage of ADA-activity causes a serious weakening in the cellular and humoral immunity (1). It is thought that the accumulation of toxic substrates and metabolites interferes with the T-cell activity and
-proliferation. The mechanism is, however, not clear at present.

ADA is widely spread in mammalian tissue where the highest levels are found in the thymus and other lymphoid tissues (2, 3). Genetic polymorphism and post translational modifications generate different physiological forms of ADA (2, 4, 5). In humans, the ADA-activity occurs substantially in two groups of iso-enzymes ADA-S and ADA-L (6). ADA-S is found in erythrocytes and consists of a single polypeptide with a molecular weight of between 36 and 44 kDa. The ADA-L-group of iso-enzymes, which are also called "tissue iso-enzymes" has a higher molecular weight and results from the interaction of the ADA-catalytic unit with a homodimeric glycoprotein of roughly 200 kDa. This glycoprotein , which is substantially membrane-bound, is called ADA binding protein (ADAbp) or ADA complexing protein (ADAcp). A native molecule of ADAbp binds two molecules of catalytic ADA. Up until now, no physiological role for ADAbp (7) has been determined. Dinjens et al. have shown that the membrane-bound ADAbp is not completely saturated with ADA-S. They suggest that due to its capability to bind ADA-S, the membrane bound ADAbp would regulate the circulation level of adenosine deaminase activity (7).

It has recently been shown that ADAbp from the human kidney is identical or analogous to the very specific ecto-enzyme dipeptidyl peptidase IV (DPP IV) (8). The molecular and biochemical characteristics and the tissue distribution of ADAbp en DPP IV appear to be inseparable (7, 9). Within the hematopoietic system, the DPP IV is, apart from this, identified as the membrane protein CD26 (10) and is substantially expressed on a subpopulation of T-cells.

It is known that, amongst other things, CD26 is related to HIV-infection (25). Furthermore the disappearance of CD26 expression is reproduced on malignant transformed melanocytes. Because CD26 is an activation marker, the CD26 expression is also a measure for determining the extent of cell activation.

From Abstract AA011of the proceedings of the 5^{th} International conference on human leukocyte differentiation antigens (November 3-7, 1993, Boston, Massachusetts) it is known that the monoclonal antibodies anti-BT5.9 and anti-TA 5.9 recognize the same or close epitope on the CD26 antigen.

De Meester et al.(Immunobiol 188, 145-158 (1993)) have disclosed that anti-BT 5/9 and anti-TA5.9 are found to react with CD 26 Ag.

It has now been found hat ligands, which bind to CD26, such as ADA itself or modified versions thereof for example, and antibodies or derivatives thereof, can have an influence on the activity or expression of CD26 and can also form a diagnostic agent for determining the CD26 expression.

Hence, for various diagnostic and therapeutic applications the provision of a ligand binding to CD26, such as for example a specific anti-CD26 antibody, is especially desirable.

The present inventors, in order to achieve this, have produced amongst other things, a monoclonal antibody which is directed against the ADA binding site of CD26 named anti-TA5.9, which is produced, for example, by hybridoma cell line anti-CD26-(TA5.9-CC1-4C8) which was deposited on 4 November 1993 at the European Collection of Animal Cell Cultures, Salisbury, GB, under the deposit accession number ECACC 93110419.

The invention thus relates to the use of monoclonal antibody Ta5.9 and functional derivatives thereof having at least one binding specificity for the adenosine deaminase (ADA) binding site of CD26 for recognition of the ADA binding site.

It has specifically been found that the anti-TA5.9 antibody has various effects.

One of the effects is that a strong co-stimulation of T-lymphocytes activity via CD3 takes place in vitro. This applies for both CD4+ (helper) and the CD8+ (cytolytic) T-cells. It is a prerequisite that the cells express CD26 on their membrane.

In the absence of soluble or immobilized anti-TA5.9 monoclonal antibody, a downmodulation of CD26 expression occurs. This downmodulation is important for the co-stimulation via CD26.

However, the membrane protein CD26 also functions as a co-receptor for the HIV-virus. Downmodulation of CD26 via anti-TA5.9 can appear to be useful here in order to prevent entrance of the virus into the target cell in vitro or in vivo.

The anti-CD26 monoclonal antibodies, with specificity for the 5.9 epitope, cause an immunosuppression which is specific for the CD26+ subset of T-cells in vivo. Such an immunosuppression can be important after transplantations and with autoimmune diseases such as multiple sclerose, rheumatism, SLE etc.

Another application of anti-TA5.9 is found in diagnosis. Malignant transformed melanocytes loose the CD26 expression on their cell membrane. By means of anti-TA5.9 it is possible to trace the disappearance of CD26 and determine the number of malignant transformed melanocytes therewith.

Anti-TA5.9 may also be used in diverse immuno-enzymatic determinations. The soluble homodimeric CD26 can be determined in an ELISA. It is also possible to detect the CD26 by means of its own enzymatic activity. Serum, plasma, seminal fluid and tissue preparations can be used as samples.

Anti-TA5.9 can be employed for determining ADA in biological fluids due to its ADA binding-site recognition characteristics.

When anti-TA5.9 is labelled with an isotope or magnetic resonance label for example, it can be used in imaging-techniques, such as scintigraphy or MRI (Magnetic Resonance Imaging). CD26 expression can be recognized in the body or in tissues with such techniques.

The above mentioned effects of the anti-TA5.9 can of course also be achieved with modified versions thereof. By modification one thinks of for example coupling to a secondary molecule selected from the group which consists of toxins, enzymes, medicines, radio active markers, fluorescent markers, magnetic resonance markers, proteases, synthetic and natural polymers.

The invention comprises apart from anti-TA5.9, furthermore all the conceivable functional derivatives thereof. By "functional derivatives" is to be understood each of the molecules derived from the antibody comprising at least one binding-site with a specificity for the adenosine deaminase (ADA) binding-site of CD26. Such a derivative may be produced by means of recombinant DNA techniques, for example, or in any other suitable way. Examples of the derivatives of the monoclonal antibody are antibody fragments such as the F_{ab}-, F_{ab'}- or F_{(ab)2}- fragment which consists of the antigen binding parts of an immunoglobulin molecule, or the Fᵥ- or scFᵥ-fragment, comprising the variable range of the immunoglobulin. The term "scFᵥ" stands for the so called "single chain" antibody which consists of the variable parts of the heavy and light chains of the immunoglobulin molecule, which are coupled to each other by means of a linker peptide which may or may not be synthetic.

By the term "functional derivatives" are also understood the combination of at least the parts of the antibody required for binding to the ADA binding-site of CD26 (named the "functional components") with at least one other molecule such as a toxin, enzyme, marker, transport protein, synthetic or natural polymer, medicines etc.

Furthermore the term "functional derivatives" comprises chimeric antibodies, such as humanized antibodies and also bispecific antibodies. The latter have two different antigen binding-sites and thereby specificities. At least one specificity is always directed towards the adenosine deaminase binding-site of CD26 while the other specificity can be directed towards any desired antigen. Here is understood other plasma membrane proteins such as CD12, CD3, CD8, CD4, markers, such as radioactive, fluorescent or magnetic resonance markers but also medicines, toxins and all other conceivable molecules which would be able to be functional in combination with CD26.

The antibody and derivatives of the invention can find a therapeutic application in the so-called "targeting". Targeting is used for bringing effector cells, medicines, toxins, imaging markers, natural and synthetic polymers etc. for example to the CD26+ cells.

A bispecific antibody which apart from the specificity for CD26 also contains a specificity for CD12, a marker for Natural Killer (NK) cells, will bring NK-activity to the target cell (CD26+). The adapted combination of CD26 and CD3 can cause proliferation of T-cells. Bispecific antibodies directed against both CD26 and CD8 can induce cytolysis of the cell concerned while due to the specificity for CD26 and CD4, the attachment and entry of HIV in target cells can be prevented.

The common characteristic of the monoclonal antibody according to the invention and all the functional derivatives thereof is the specificity for the ADA binding-site of CD26.

The invention shall further be exemplified by reference to the following examples which are not however meant to limit the invention in any way.

### EXAMPLE 1

### ADA-binding on the lymphocyte surface

### 1. Introduction

Some monoclonal antibodies which are specific for CD26, exhibit co-stimulating activity for TcR/CD3 induced T-cell activity (11, 12). De DPP-IV-activity of CD26 plays an important role, not only in CD26 mediated co-stimulation, but also in IL-2-production after different stimuli (13-15). The expression of CD26 on the surface (16) and the specific DPP-IV-activity (17) is increased after T-cell-activation. The functional importance of the CD26 antigen and its DPP-IV-activity in the T-cell-activation cascade, together with the apparently essential role of ADA in the development of normal immune responses, has brought the inventors to examine different aspects of the interaction between ADA and the lymphocytic CD26/DPPIV. In this way the binding of the monoclonal antibody anti-TA5.9 according to the invention, which is directed against CD26, and a few other anti-CD26 monoclonals, to different lymphocyte subsets has been compared with the surface expression patterns of ADAbp. Apart from this, the influence of ADA on the interaction between CD26 and the monoclonal antibodies, which recognize differing epitopes on the CD26, were studied. Furthermore, an analysis was carried out as to the effect of ADA on DPP-IV-enzyme activity. Moreover, the direct binding of pure CD26/DPP IV to immobilized ADA was proved. Finally the influence of ADA on CD26 surface expression was analyzed.

### 2. Materials and methods

### 2.1 The preparation of monoclonal antibody anti-TA5.9.

The monoclonal antibody anti-TA5.9 is produced by the hybridoma cell line anti-CD26-(TA5.9-CC1-4C8), which on November 4, 1993 was deposited at the European Collection of Animal Cell Cultures, Salisbury, GB, under the deposit accession number ECACC 93110419. The hybridoma cell line, here after referred to with the name LY12.CC1, was obtained in the manner described below. Information about these known techniques may be found in J. Eryl Liddell & A. Cryer, "A practical guide to monoclonal antibodies", John Wiley & Sons (1991), for example.

The immunization took place by the intraperitoneal injection of BALB/c-mice with PHA stimulated human peripheral blood mononuclear cells. Complete cells were used suspended in PBS, without the addition of an adjuvant.

The hybridomas were produced by fusing spleen cells of the immunized mice with SP2/0-AG14 myeloma cells using PEG as the fusing promotor. The hybridomas were selected by growing them on HAT-medium.

Thereafter the hybridomas were screened on the production of antibodies which were specific for human mononuclear cells activated with PHA. All the screening tests were carried out by means of flow-cytometry. In the first screening test, the antibody reactivity of the cells stimulated with PHA was measured. Antibodies which exhibited a strong reactivity in this first screening were again tested in the second screening test for binding to resting human peripheral blood mononuclear cells. Antibodies which exhibited a significantly higher reactivity with the PHA stimulated cells in comparison with resting cells, were selected for further development.

The selected hybridoma CC1 was twice cloned by limited dilution. Microtiter wells which contained a single growing cell colony were retested in the same screening procedure. Clone Ly12.CC1 was chosen for further proliferation and antibody production. The antibodies were produced in the ascites liquid of the BALB/c-mice which were injected with cells from the Ly12.CC1 clone. By means of affinity chromatography on protein A Sepharose, monoclonal antibody was purified from the ascites fluid.

The binding characteristics of the monoclonal antibody Ly12.CC1 with different types of cells, was tested by flowcytometry. The antibody exhibited a high reactivity with all the tested preparations of human peripheral blood mononuclear cells stimulated with PHA. Up to 98% of these cells reacted positively. On the contrary, resting human peripheral blood mononuclear cells generally exhibited a weaker reactivity with this antibody, while a clear differentiation between the populations of a positive of negative reacting cells was detected. Furthermore, simultaneous two color cytometric analysis showed that the positive reacting cell population of the resting mononuclear cells, consisted of a subpopulation of T-cells including CD4 positive an CD8 positive T-cells. Similar studies showed that this antibody did not react with the T-cell activation markers CD25 (IL2-receptor), CD71 (transferrin receptor) or HLA-DR. Subtyping disclosed that the antibody was a mouse IgG1 monoclonal antibody.

### 2.2 Cell preparation and FCM-analysis

Human Peripheral Blood Mononuclear Cells (PBMC) were isolated from blood made oncoagulatable with EDTA, by density gradient-centrifugation on Ficoll-Paque, such as described earlier (18). The PBMC were harvested for 4 days on a density of 0,5 x 10⁶/ml in RPMI-1640-medium suppleted with 10% FCS, glutamine and antibiotic (further called "complete medium") in the presence of 0.5 µg/ml PHA (phytohemagglutinin, purified quality from Wellcome, Dartford, England).

Indirect immunofluorescence-staining of washed PBMC, which were harvested or originated from complete blood, was carried out in PBS with 1% BSA, 0.05% sodium azide, using the anti-CD26 antibodies anti-Ta1 (Coulter, Hialeach), anti-BT5.9 (obtained from Dr. Corte, Genua, Italy) and the anti-TA5.9 according to the invention. Apart from this, the anti-ADABp-monoclonal URO-4 (clone S-27) from Signet Laboratories, Dedham, MA, was used. After incubation of the specific monoclonal antibodies for 20 minutes at 4°C, the cells were twice washed and the F(ab')₂ (produced in goat en directed against mouse IgG) that was supplied with FITC or PE (Tago, Burlingham, Ca) was added during an other 20 minutes at 4°C. In the case of complete blood samples, the red blood cells were lysated before washing and fixating of the cells. The samples were analyzed on a FACScan flow cytometer (Becton Dickinson, Erembodegem, Belgium). For direct immunofluorescence, FITC and PE conjugated in monoclonal antibodies from Becton Dickinson (apart from anti-TA5.9-FITC) were used. The samples were incubated and lysated for 20 minutes at 4°C, washed and fixed as described above. As a control, iso-type monoclonal antibodies with a known specificity were used.

Two color experiments with one non-conjugated and one conjugated specific monoclonal antibody were carried out as follows. The samples were incubated (20 minutes, 4°C) with a non-conjugated monoclonal antibody and washed. F_{(ab')} (produced in goat directed against mouse-IgG) labeled with FITC or PE was added thereto during a further 20 minutes at 4°C. After washing the free binding-sites on cell bound goat-anti-mouse-fragments were blocked with 5 µl of mouse serum. Finally the directly conjugated monoclonal antibody was added for a last incubation and washing cycle. The samples were treated as described above.

### 2.3 Influence of ADA on FCM-analysis and expression of lymphocytic CD26/DPP IV.

The influence of ADA on the binding of anti-CD26 monoclonal antibodies on the lymphocyte surface was tested by pre-incubation of stimulated PBMC or washed complete blood with differing amounts of bovine adenosine deaminase (type V, Sigma, Bornem, Belgium) for 30 minutes at room temperature in PBS with 1% BSA and 0.05% sodium azide. Thereafter the cells were twice washed with the same buffer. Following this, indirect or direct staining of the cells was carried out as described above.

In order to study the downregulation of CD26 expression on the surface by ADA, the PBMC were freed from contaminating monocytes (by attachment to polystyrene plates) and incubated in complete medium at 37°C for different periods of time (2, 4, 8 and 24 hours) in the presence or absence of bovine-ADA (0.5 to 50 µg/ml) or anti-TA5.9 according to the invention (0.5 to 50 µg/ml). In a first series of experiments, ADA and the anti-TA5.9 monoclonal antibody were added in solution, and in a second series they were immobilized on polystyrene plates. After incubation, the samples were cooled on ice and indirectly stained with anti-Ta1 monoclonal antibody as described above.

### 2.4. Interaction of ADA with purified CD26 dipeptidyl peptidase IV.

In order to study the influence of ADA on the enzymatic activity of DPP IV, bovine-ADA (0.02-1.7 µg/ml agree with 2-180 mU/ml) was incubated for one hour at 37°C with purified human dipeptidyl peptidase IV. Thereafter the DPP IV-activity was measured with using the fluorogenic substrate Gly-Pro-4-Methoxy-2-Naphtylamide such as described elsewhere (19). In order to study the effect of ADA on the interaction between purified DPP IV and immobilized monoclonal antibodies (20), anti-CD26 monoclonal antibodies (anti-Ta1, anti-TA5.9 according to the invention, anti-BT5.9, 134-2C2 and anti-1F7 were spotted directly onto a strip of nitrocellulose. The 134-2C2 and anti-1F7 monoclonal antibodies were obtained from Dr. Plana, Barcelona, Spain and Dr. Morimoto, Boston, MA resp. After blocking and washing such as described in (20) the monoclonal antibody containing strips were incubated at 4°C with DPP IV which had been previously incubated with bovine ADA (1 hour at 37°C). In blanc and control experiments, the strips were incubated in PBS-buffer or in DPP IV without ADA, respectively. After incubation overnight, the membranes were washed twice with 20 mmole/1 Tris-HCl, pH 7.4 with 1 mole/1 NaCl and the DPP IV bound was fluorometrically measured as described in (20).

The interaction between ADA and purified dipeptidyl peptidase IV was further investigated using affinity chromatography. ADA from calf intestines (type VI, Sigma) was coupled to CNBr-Sepharose 6B (Sigma) in a concentration of 5 mg/ml gel, as described by the supplier. The columns were equilibrated with 20 mmole/1 Tris-HCl, pH 7.4 with 0.5 mole/l NaCl in order to avoid a-specific binding. Preparations of purified DPP IV (100 mU), alone or previously incubated (1 hour at room temperature) with an excess of mouse-IgG1 with a non relevant specificity, anti-Ta1 monoclonal antibody, URO-4 monoclonal antibody or anti-TA5.9 monoclonal antibody according to the invention, were mounted on to the ADA-Sepharose column. The binding of CD26/DPP IV was measured by determining the DPP IV activity in the non-bound fraction. In order to control a-specific interactions with the carrier, the same preparations were loaded on blocked CNB4-Sepharose 6B.

### 3. Results and discussion

### 3.1 The binding profiles of anti-ADAbp monoclonal antibodies and anti-CD26 monoclonal antibodies on lymphocyte subsets are analogous.

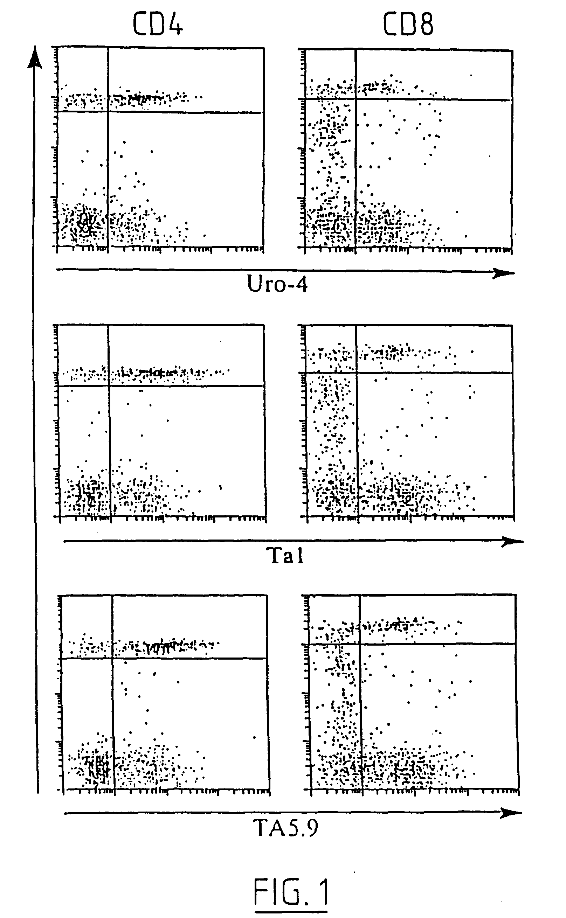

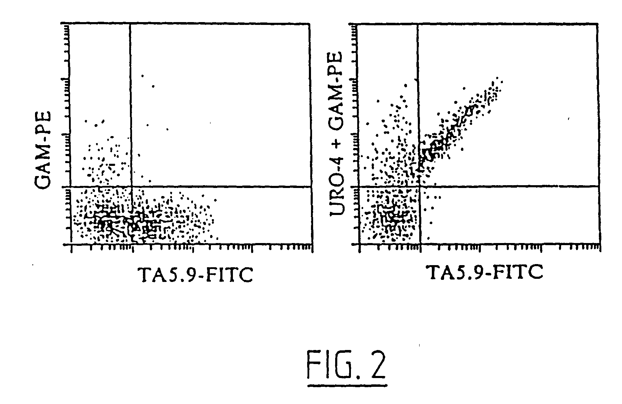

The monoclonal antibody URO-4, produced against a human cancer kidney cell line, recognizes the adenosine deaminase binding protein from human proximal tubular cells, fibroblasts and HepG2 cells (21, 22). In order to further examine the reported analogy or identity between ADAbp and CD26, the monoclonal antibody URO-4 was introduced into flow cytometric analyses of complete blood or PBMC. Both sources gave similar results. As demonstrated in figure 1, the reactivity patterns of URO-4 on different lymphocyte subsets (CD4+ and CD8+ lymphocytes and (not shown) CD19+ and CD16/56+ lymphocytes) were completely comparable to those obtained with anti-CD26 monoclonal antibodies anti-Tal and anti-TA5.9 according to the invention. With the antibody anti-TA5.9, information about the CD26 expression on T-cell subsets was obtained. Roughly 50% of the CD5 positive cells appeared to express membrane-bound ADAbp (23). Simultaneous staining with URO-4 and anti-TA5.9 monoclonal antibodies showed that both antibodies bind to the same cells (fig. 2). This indicates that ADAbp and CD26/DPP IV are identical.

### 3.2 ADA blocks the binding of anti-TA5.9, but not that of anti-Ta1, to CD26 on the surface of the lymphocytes.

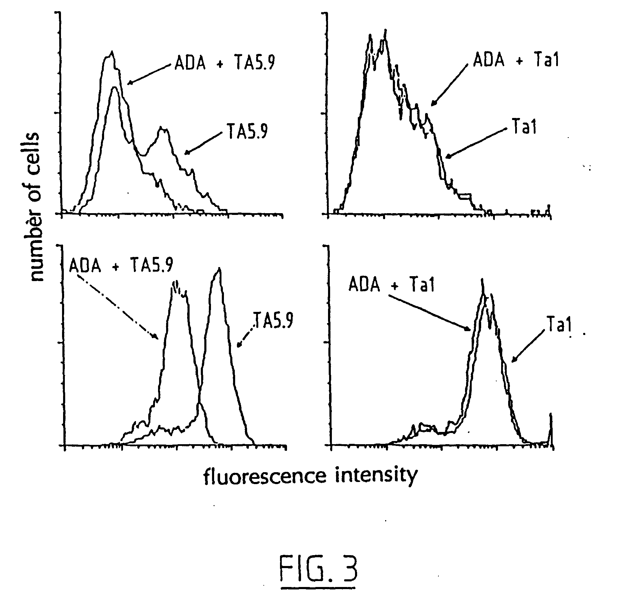

Fig. 3 shows the influence of ADA on the fluorescent profile of anti-TA5.9 and anti-Ta1 monoclonal antibodies. ADA strongly inhibits the binding of anti-TA5.9 to the surface of both resting and stimulating lymphocytes. The binding of anti-Ta1 and URO-4 monoclonal antibodies was, however, not reduced by ADA (results not shown). The results indicated that ADA binds to or near the TA5.9 epitope of CD26. The Ta1 epitope (of which it is reported that it reacts with the HIV-1 regulation protein TAT (24)) did not appear to be related with ADA binding to the lymphocyte surface. Earlier observations that the binding of ADA to kidney ADAbp did not inhibit the immunoprecipitation of ADAbp by URO-4 (21) are in accordance with our experiments where no influence of ADA on the binding of URO-4 to lymphocyte CD26 was detected.

### 3.3 ADA strongly reduces the binding of purified DPP IV to immobilized anti-TA5.9, but not to anti-IF7 and anti-Ta1 anti-CD26 monoclonal antibodies.

Apart from the flowcytometric analyses, binding studies were carried out on the soluble DPP IV and DPP IV/ADA-complexes and the immobilized monoclonal antibodies such as outlined out in Materials and Methods. Complex-forming with ADA destroyed the binding of DPP IV and anti-TA5.9 almost completely, whilst a slight or no effect was seen on the binding to anti-Ta1 and anti-IF7 monoclonal antibodies (data not shown). These results confirm that anti-TA5.9 is specific for the epitope on CD26, which is related to ADA-binding.

### 3.4 Anti-TA5.9 inhibits the binding of purified CD26/DPP IV to immobilized ADA.

The affinity chromatography experiments directly showed the interaction of CD26/DPP IV with ADA and the relation of the TA5.9-epitope in the binding between these two molecules. When CD26/DPP IV was alone loaded on an ADA-Sepharose 6B, 100% of the enzyme was binding to the column. CD26/DDP IV preparations which were incubated with anti-Ta1 or URO-4 monoclonal antibodies, were both binding for more than 85%. However, the anti-TA5.9 monoclonal antibody lowered the binding of CD26/DPP IV to immobilized ADA strongly to less than 20% of the quantity produced. This phenomenon was not caused by the a-specific binding of free anti-TA5.9 monoclonal antibody to ADA because in a control experiment 100% of the FITC labeled anti-TA5.9 monoclonal antibody on a ADA-Sepharose 6B column was found in the non bonded fraction.

### 3.5 ADA did not inhibit the DDP IV enzymatic activity.

In order to be confirm and extend the finding on the binding of ADA to CD26/DPP IV the effects of ADA on DPP IV enzymatic activity were studied. No decrease in Gly-Pro-4-methoxy-2-naphtylamide hydrolysis was found in DPP IV after incubation with varying concentrations of ADA (results not shown).

### 3.6 ADA does not modulate the CD26 surface

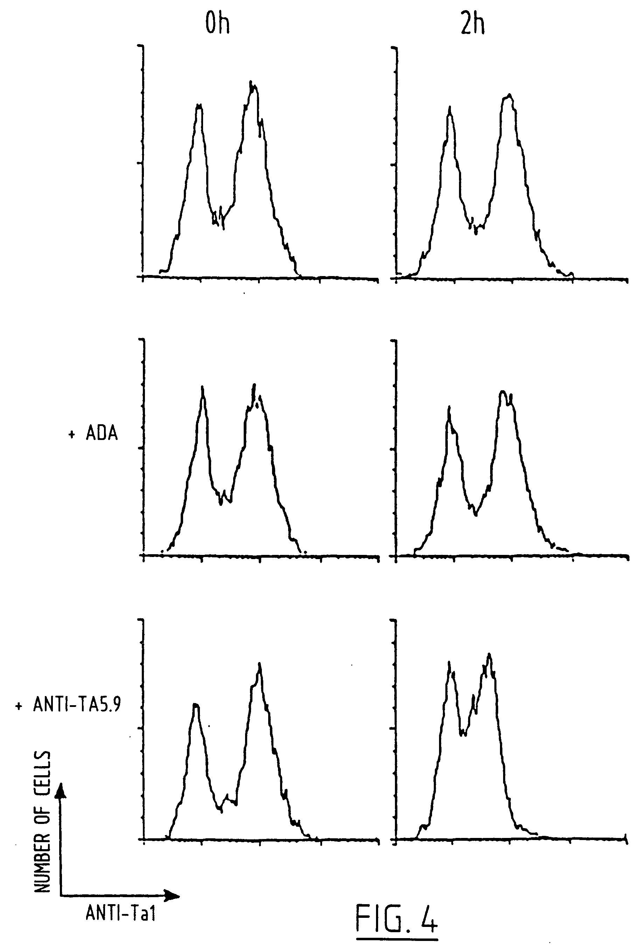

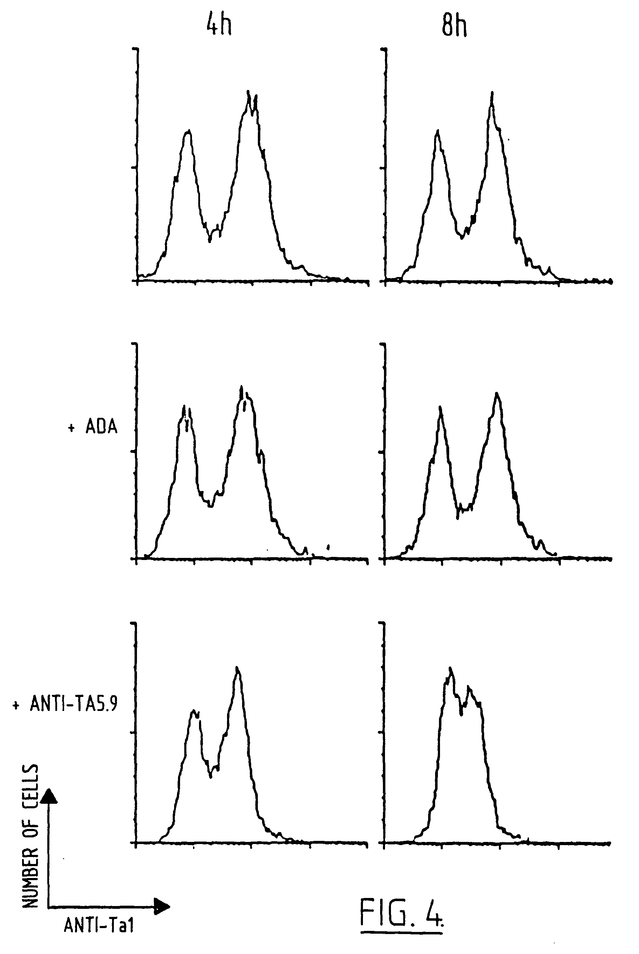

The ability of ADA to cause downregulation of CD26 expression on the cell surface, was invetsigated by analysis thereof after incubation of cells with varying concentrations of ADA for differing time intervals. Modulation of CD26 by anti-TA5.9 was carried out as a positive control. The results are shown in fig. 4.. After incubation with anti-TA5.9, the expression of CD26 on the surface began to decrease within two hours incubation time. High concentrations of anti-TA5.9 monoclonal antibody induced a quicker reduction in CD26-expression. ADA was not capable, under any of the given circumstances, to modulate CD26-expression on the T-cell surface. An anti-Ta1 monoclonal antibody was used to detect CD26 on the cell surface, because the binding of ADA masks the TA5.9 epitope, but not the Ta1 epitope of CD26.

Until now, none of the possible ligands and/or substrates for CD26/DPP IV showed that they could induce the downregulation of CD26 surface expression. Anti-TA5.9 does this.

### 4. Conclusion

The ecto-enzyme DPP IV, which is identical to lymphocytic CD26 is also the ADA binding protein of these cells. This is an intriguing observation considering the known co-stimulatory effects of anti-CD26 monoclonals and considering the serious immune damage caused by ADA deficiency. A critical site for ADA binding to CD26/DDP IV which is selectively related to the epitope, which is recognized by the monoclonal antibody according to the invention anti-TA5.9 was found. The results reproduced here further show that ADA, when bound to DDP IV, forms a type bifunctional enzymatic complex that may be active in the bloodstream and in tissues. Under the experimental conditions used, ADA was not capable of modulating CD26 on the lymphocyte surface. This was surprising considering that the anti-TA5.9 monoclonal antibody which reacted with a very closely related or identical epitope, quickly downregulated the CD26-expression.

### 5. Description of the figures.

Figure 1 shows the binding of the anti-CD26 and anti-ADAbp monoclonal antibodies on CD4+ (left column) and CD8+ (right column) lymphocytes. Whole blood was indirectly stained with the anti-ADAbp monoclonal antibody URO-4 (at the top), or the anti-CD26 monoclonal antibody anti-Ta1 (in the middle) and anti-TA5.9 according to the invention (below) . In all cases FITC-labeled F_{(ab')2} produced in goat and directed against mouse-IgG was used as the secondary reagent. Anti-CD4-PE (left column) or anti-CD8-PE (right column) was used as the second marker.
Figure 2 shows the co-expression of anti-CD26 and anti-ADAbp on lymphocytes. Whole blood was stained with either PE-labeled F_{(ab')2} alone produced in goat and directed against mouse-IgG (left) or URO-4 and PE-labeled F_{(ab')2} produced in goat and directed against mouse-IgG. In both cases anti-TA5.9 was used as the secondary reagent.
Figure 3 shows the influence of ADA on the binding of anti-TA5.9 (left) and anti-Ta1 (right) on the surface of resting PBL (above) or PHA-stimulated PBL (below). 2 x 10⁵ cells were incubated in the presence or absence of ADA (6 µg/ml) for 30 minutes at room temperature. Thereafter, the binding of anti-TA5.9 and anti-Ta1 to the lymphocyte surface was examined by indirect FCM.
Figure 4 shows that anti-TA5.9, but not ADA, induces the downmodulation of CD26. PBL was incubated for the given time intervals (0, 2, 4 and 8 hours) in complete medium at 37°C, as such (above) in the presence of ADA (5 µg/ml) or the absence of anti-TA5.9 (5 µg/ml) (below). Thereafter CD26 was visualized on the cell surface by FCM and using the anti-Ta1 and PE-labeled F_{(ab')2} produced in goat and directed against mouse-IgG.

### EXAMPLE 2

### Diagnostic determinations

### 1. Introduction

Hereinbelow are described two assays for the determination of CD26 in serum en seminal plasma. On the one hand that is a fluorometric assay based on the protease-activity of DPP IV and on the other hand it is a sandwich-ELISA. The first step in both tests is based on the capture of CD26 by the monoclonal antibody anti-TA5.9 according to the invention. The fluorometric assay is a functional determination measuring the proteolytic activity of the captured CD26 with a fluorogenic substrate. The ELISA determines the immunoreactive quantity of the soluble homodimer CD26.

### 2. Fluorometric assay for the determination of CD26.

### 2.1 Introduction

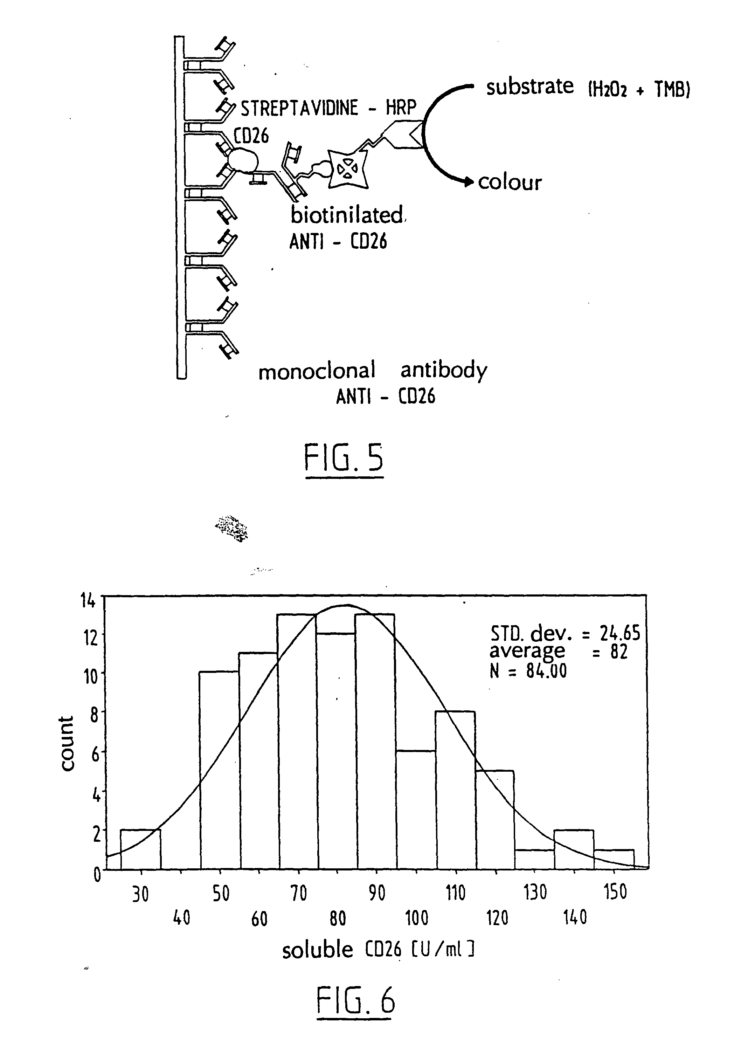

In the fluorometric assay, the activity of CD26 in serum or seminal fluid is measured. The CD26 is captured by the anti-TA5.9 applied to an adapted carrier. After washing away the unbound material, a fluorogenic substrate Gly-Pro-7-amino-4-methoxy coumarin is added and split by the bound CD26 in order to free a fluorescent molecule, in this case 7-amino-4-methoxy-coumarin. This last molecule can be directly measured (see fig. 5).

### 2.2 Method

Non-transparent microtiter plates of Nunc (Denmark), were covered with anti-TA5.9 (overnight at 4°C in a carbonate buffer, pH 9.6, 3 µg/ml). Remaining free binding sites, were blocked the following morning with 3% BSA in PBS pH 7.2. A pool of 10 different seminal fluids was made as a reference standard and dissolved in human albumin matrix (SOPP, Belgium Red Cross). The DPP IV activity of this reference standard was determined according to the method described in reference 19.

The reference standard was diluted in SOPP to 25 mU/ml, 2.5 mU/ml and 0.5 mU/ml. A series of dilutions was made from the seminal liquid from 100 x to 10.000 x.

100 µl of standard and test samples were placed in the wells of the microtiter plates. Incubations were carried out for two hours. A substrate stock solution of Glyco-Pro-7-amino-4-methoxy coumarin (20 mmol/l in DMSO) was diluted 12 times in incubation buffer (50 mM Tris-HCl, pH 8.3). Thereafter the plates were washed 5 times with PBS pH 7.2 buffer with Tween 80. Following this, 100 µl of the diluted substrate solution was added to each well. Incubation was then carried out for one hour at 37°C. The reaction was stopped by the addition of 100 µl 1M citrate pH 4.0 to each well. The intensity of the fluorescence signal was determined in a Fluoskan instrument at 355 nm and 425 nm.

### 2.3. Results

The results of a population study, carried out on a series of 48 healthy blood donors with the fluorometric assay for CD26, are represented in fig. 5.

### 3. ELISA for the detection of CD26

### 3.1. Introduction

Microtiter plates were coated with the anti-TA5.9 monoclonal antibody for capturing CD26 from the sample. After washing away the non-bound material, the bound homodimer CD26 was detected by biotinylated anti-TA5.9 monoclonal antibody and streptavidin-HRP. The HRP (horseradish peroxidase) reacted with H₂O₂ and the chromogenic tetramethylbenzidine (TMB). This resulted in a blue reaction product which underwent a change of color to yellow after stopping the substrate reaction with 2M H₂SO₄. See fig. 6.

### 3.2. Method

The reference standard (see under 2.2) was diluted in SOPP to 25 mU/ml, 2.5 mU/ml and 0.5 mU/ml. The seminal fluid, which was used as the test sample, was serially diluted from 100 x to 10.000 x. 100 µl of standard- and test samples were placed in the wells and incubated for two hours at 37°C. Thereafter the plates were washed 5 x with a wash solution (PBS-buffer, pH 7.2 with 82). 100 µl of the biotinylated conjugate was then added to each well. The biotinylated antibody was diluted in TBS-buffer, pH 7.2 with 0.1% Tween 20 and 2.5% BSA. The buffer in which the biotinylated monoclonal antibody is diluted, contains then 25 µg/ml of mouse-IgG1. After an incubation of 1 hour at 37°C, the plates are washed 5 x with wash solution. Thereafter 100 µl streptavidin-HRP is added to each well. The streptavidin is diluted in the same way as the biotinylated antibody. After an incubation of 20 minutes at 37°C, the plates are washed 5 x with wash solution, after which 100 µl of H₂O₂ substrate solution and 100 µl of chromogenic solution (TMB, tetramethyl benzidine) is added to each well. After an incubation of 20 minutes at 37°C the reaction is stopped by addition of 50 µl stopping buffer. Finally the absorption was determined at 450 nm.

### 3.3. Results

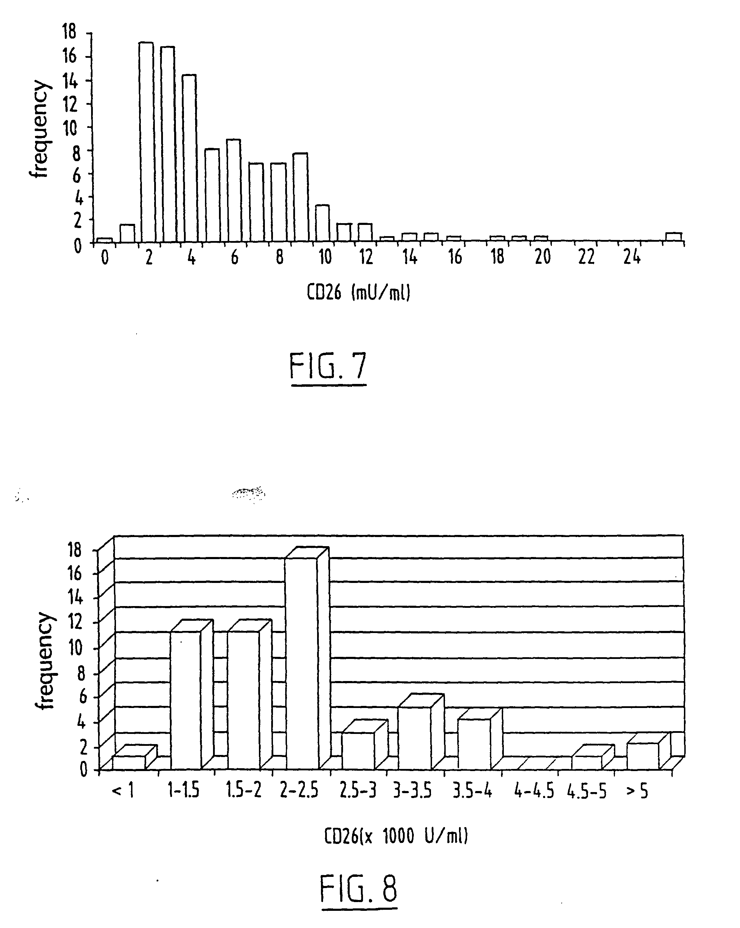

The results for 245 serum samples of healthy blood donors between 18 and 65 years, were analyzed. The results are shown in the graph of figure 7.

Apart from this 55 seminal plasma samples were analyzed. The results are shown in the graph of fig. 8.

### 4. Enzyme-immunoassay (EIA) for determining adenosine deaminase in biological fluids.

### 4.1. Introduction

Due to the specific recognition of ADA-binding sites on CD26 by TA5.9, the monoclonal can, in combination with ADA-free CD26, which, either directly or via immunocapatation is immobilized onto a solid carrier, be used for determining adenosine deaminase in biological fluids by means of an inhibition test.

### 4.2. Method

Transparent polystyrene microtiter plates (Nunc, Denmark) are coated overnight at 4°C with anti-CD26 by incubation with a solution of 3 µg/ml anti-TA5.9 in carbonate buffer, pH 9.6. The remaining free binding sites are blocked the next morning with 3% BSA.

A reference preparation, which contains 50 mU/ml CD26, was prepared in a human albumin matrix SOPP (Belgian Red Cross). A series of standards was also made with bovine adenosine deaminase EC3.5 4.4 (Sigma A-1155) which contained 0 U/ml, 1 U/ml, 10 U/ml and 100 U/ml ADA, respectively.

The inhibition EIA is carried out as follows. In a first step, 100 µl of the ADA-standards (0,1, 10 and 100 U/ml) are placed in the wells of the microtiter plate. Incubation is carried out for one hour at 37°C. Thereafter the plate is washed 5 x with PBS-buffer, pH 7.2. In a second step 100 µl of a biotinylated TA 5.9 conjugate, which is diluted in PBS-buffer pH 7.2, is added with 0.1% Tween 20 and 2.5% BSA. After incubation for one hour at 37°C, the plates are washed 5 x with washing buffer. Thereafter 100 µl of streptavidin-HRP is added to each well. The streptavidin-HRP is diluted in the same way as the biotinylated antibody. After incubating for 20 minutes at 37°C the plates are again washed 5 x with washing solution after which 100 µl of a H₂O₂ substrate solution and 100 µl chromogenic reagent (TMB) is added to each well. After incubating for 20 minutes at 37°C the reaction is stopped by the addition of 50 µl 2 N H₂SO₄. Finally the optical density of the solution is determined at 450 nm.

### 4.3. Results

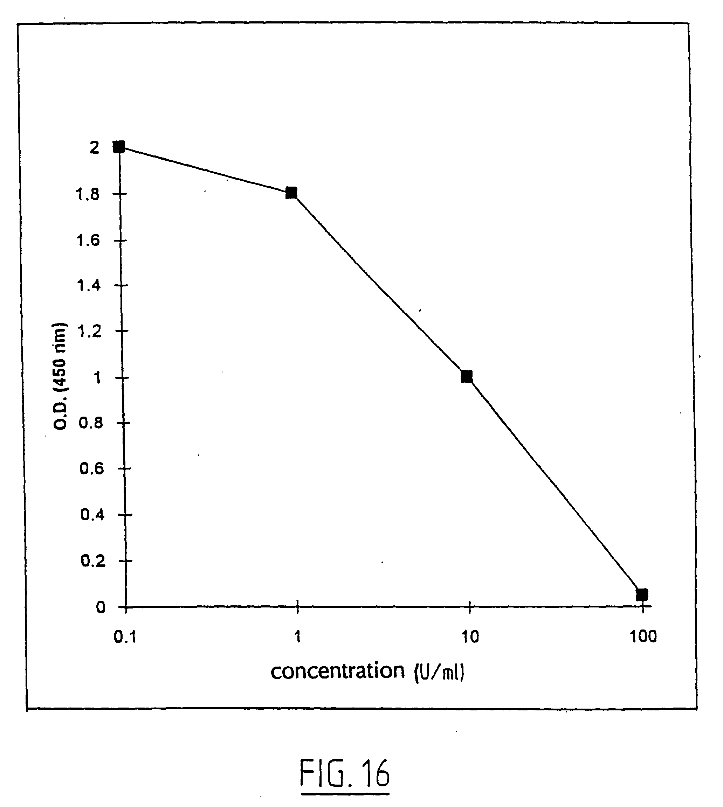

The results of a standardization example of an ADA-concentration assay by means of inhibition EIA are represented in figure 16.

### EXAMPLE 3

### In vitro co-stimulating properties of the anti-CD26 monoclonal antibody anti TA5.9

### 1. Introduction

Various plasma membrane proteins are related to T-cell activation. It is known that apart from the classical T-cell receptor-CD3-complex, dependending on the experimental conditions, the triggering of CD2, CD5 and CD28 can contribute to T-cell activation. It is suspected that a co-mitogenic effect for the stimulation of 004+ T-cells also exists for anti-CD26 monoclonal antibodies. In this example this effect of the anti-TA5.9 monoclonal antibody according to the invention on CD26-CD3 mediated activity of purified CD4+ and CD8+ T-cells is investigated. This way it is possible to compare the in vitro effects of anti-TA5.9 on CD4+ T-cells with other anti-CD26 monoclonal antibodies and to investigate the effect of CD26 triggering on CD8+ T-cells.

### 2. Method

The activation and proliferation of CD4+ and CD8+ T-cells were monitored via determining DNA-synthesis on the one hand and the expression of the activation markers on the cell surface on the other hand.

Human CD4+ and CD8+ T-cells were purified from peripheral blood (EDTA as anti-coagulant) by isolating PBMC via Ficoll-Paque density gradients, removing monocytes by adhering them to polystyrene petri-dishes (for 2 hours at 37°C), the positive selection of CD8+ T-cells by means of an immuno-magnetic carrier and the positive selection of CD4+ T-cells from the remaining cell population, also via an immuno-magnetic carrier. The purified cell populations from each donor were stimulated parallel and under identical conditions in the presence of the following immobilized stimuli:
(1) Control monoclonal antibody (anti-MHC class I monoclonal antibody)
(2) anti-CD3 (OKT3) monoclonal antibody and control monoclonal antibody
(3) anti-CD26 monoclonal antibody (TA5.9 or TA1)
(4) anti-CD3 and anti-CD26 monoclonal antibody (TA5.9 or TA1)

The expression of the activation marker CD69, CD25, CD71 and HLA-DR on CD4+ and CD8+ T-cells was determined at different moments during the activation. Their CD45RO+ (memory) and CD45RO- (naive subsets) were also determined flow cytometrically (FACScan). The cell proliferation was measured by means of ³H-thymidine incorporation (in quadruplicate) after 5 days. The cell populations obtained have an average purity of 96% for the CD4+ T-cells and 95% for the CD8+ T-cells.

### 3. Results

The anti-CD26 monoclonal antibodies Ta1 and TA5.9 cannot produce any proliferation on their own. The immobilized anti-CD3 alone in some individuals caused a significant proliferation of CD8+ T-cells but never of CD4+ T-cells. A co-stimulation via CD26 (TA5.9 or Ta1) involves a strong proliferation of CD4+ and CD8+ T-cells. Cyclosporin blocks the T-cell proliferation which is caused by the CD3-CD26-co-stimulation.

The expression of the activation markers on CD4+ and CD8+ T-cells is as follows:

Anti-CD3 induced a significant expression of CD69 on purified CD8+ T-cells, whilst for the CD4+ T-cells, only a small and late increase is to be seen. In a similar manner, but less clearly, the activation markers CD25, and in some individuals also CD71, are expressed on CD8+ T-cells but not on CD4+ T-cells after anti-CD3 stimulation. An important co-stimulating effect of anti-CD26 monoclonal antibodies (Ta1 and TA5.9) is observed on the CD3 mediated activity. The expression of CD69 on CD4+ and CD8+ T-cells is within 24 hours after the start of this co-stimulation at the most. The expression of CD25 and CD71 also increases to more than 90% positive cells after 72 hours and this is also the case for the CD4+ and the CD8+ T-cells.

Considering the expression of activation markers on CD45RO+ and RO- subsets of CD4+ and CD8+ T-cells, the following has been found. After stimulation of CD8+ T-cells with anti-CD3, CD69 is expressed on both the CD45RO+ and CD45RO- subsets. This expression is however twice as high on the CD45RO+ CD8+ T-cells in comparison to the CD45RO- CD8+ T-cells. CD25 is mainly expressed on CD45RO+ CD8+ T-cells. Co-stimulation via CD26 results in a comparable increase of CD69 and CD25 expression both on memory and naive CD4+ and CD8+ T-cells. The up-regulation of CD25 occurs much quicker on the CD45RO+ cells.

Another much used parameter of T-cell activity is the expression of HLA-DR. Under control conditions the HLA-DR within the CD4+ T-cells is almost exclusively present on the CD45RO+ subsets. Within the CD8+ cell population HLA-DR is mainly, but not exclusively, present on the CD45RO+ cells. The up-regulation of HLA-DR expression on the CD4 and CD8+ T-cells after CD3-CD26 co-stimulation, occurs quicker and is more apparent on the CD45RO+ subset of both T-cell populations.

### 4. Conclusion

It can generally be assumed that a strong co-stimulating effect is observed of anti-CD3 and anti-CD26 monoclonal antibodies on purified CD4+ and CD8+ T-cells. The expression of activation markers is more accelerated on CD8+ in comparison with CD4+ T-cells and on CD45RO+ with respect to CD45RO- subsets. The co-stimulatory effect of anti-TA5.9 is in general clearly higher than that of anti-Ta1.

### 5. Description of the figures

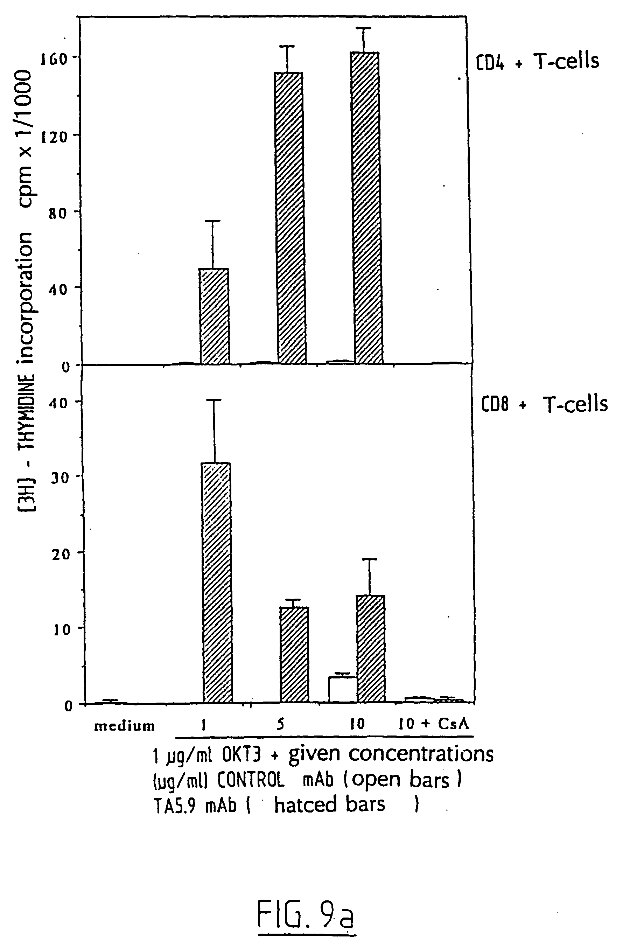

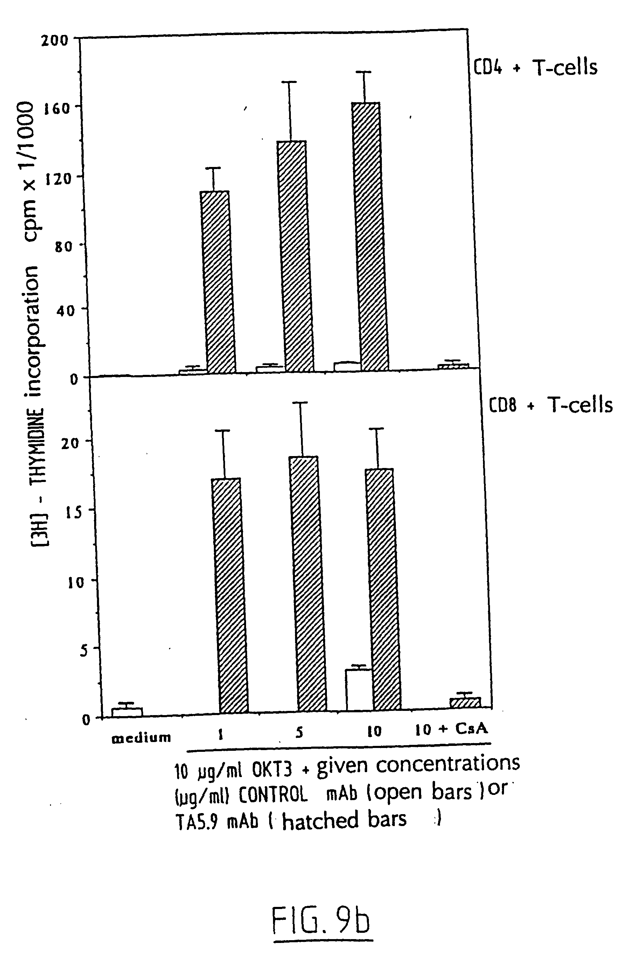

In figure 9a the co-stimulation of purified CD8+ and CD4+ T-cells by 1 µg/ml immobilized anti-CD3 (OKT3) in combination with a control monoclonal (open blocks) or anti-TA5.9 (hatched blocks) respectively, is shown. Figure 9b gives result for 10 µg/ml OKT3.

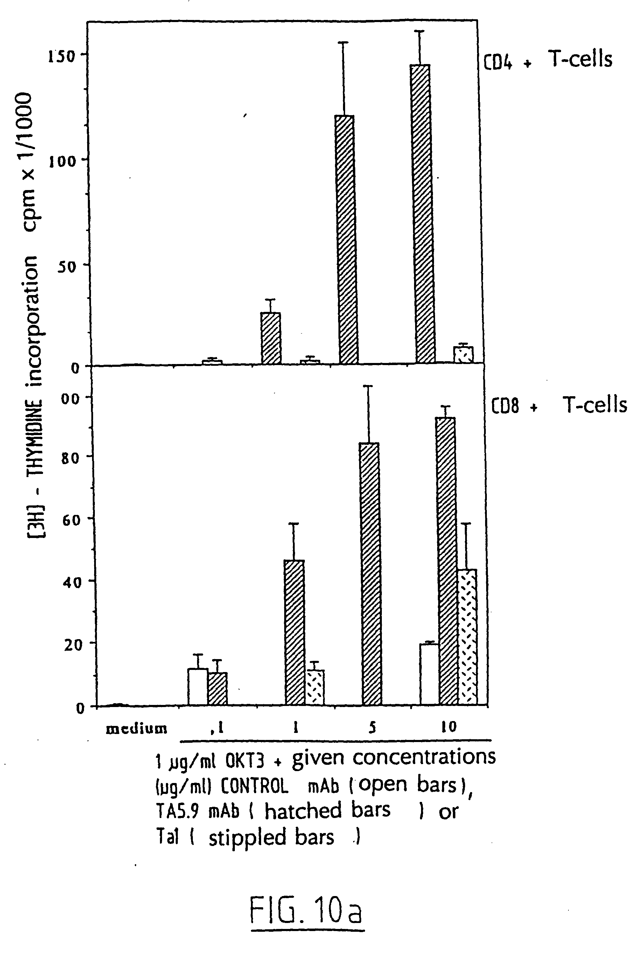

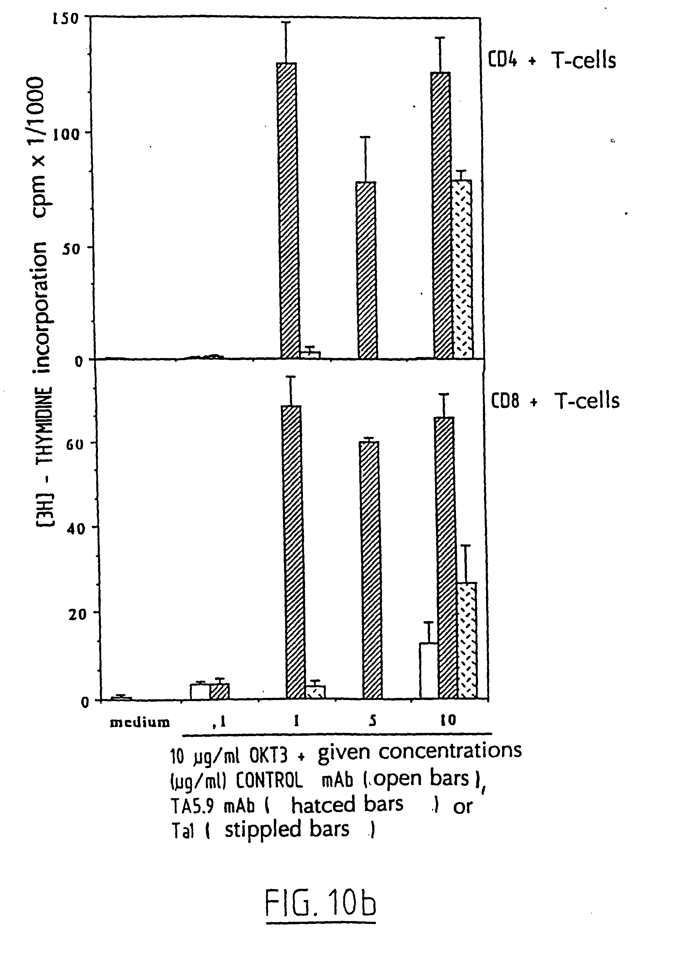

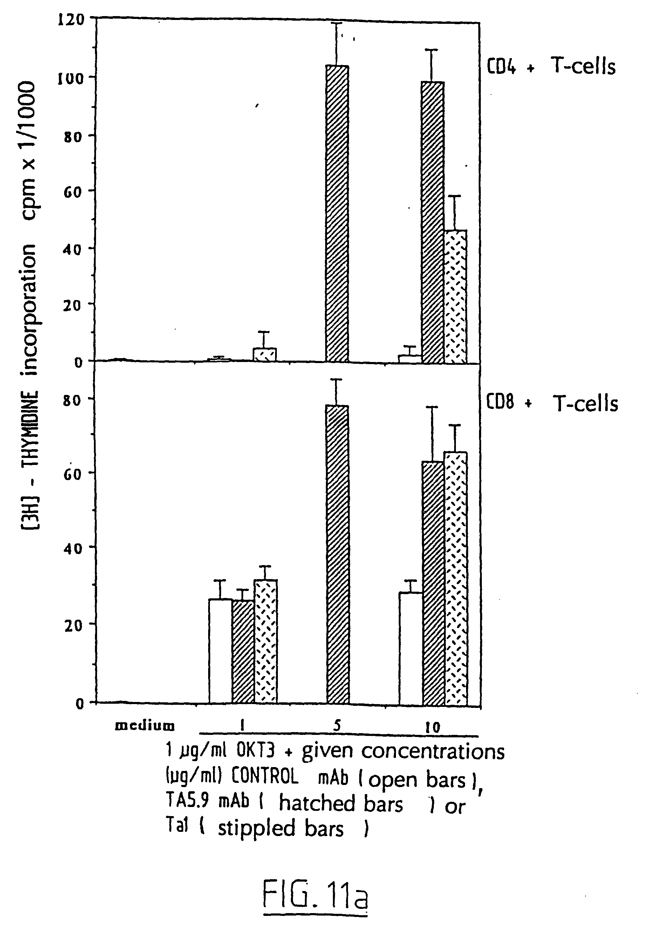

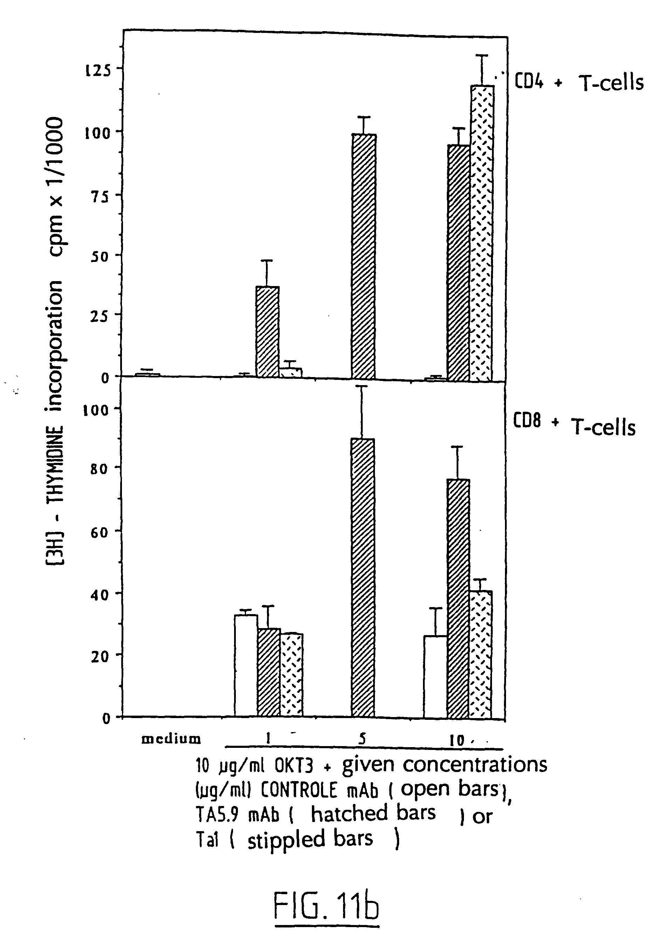

Figures 10 and 11 give again the co-stimulation of purified CD4+ and CD8+ T-cells by 1 µg/ml (a) and 10 µg/ml (b) immobilized anti-CD3 (OKT3) respectively in combination with a control monoclonal (open blocks), anti-TA5.9 (hatched blocks), or anti-Ta1 (stippled blocks) respectively.

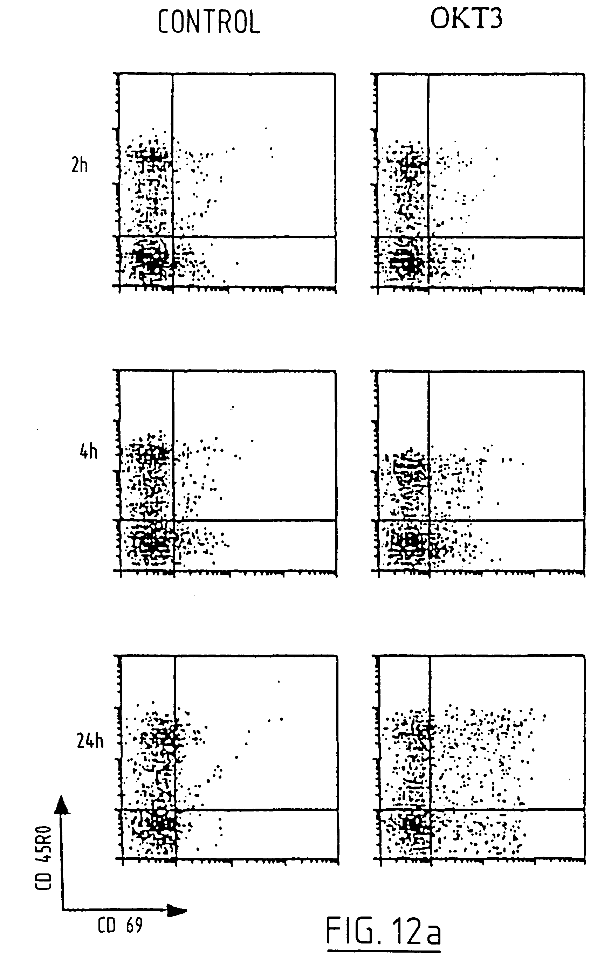

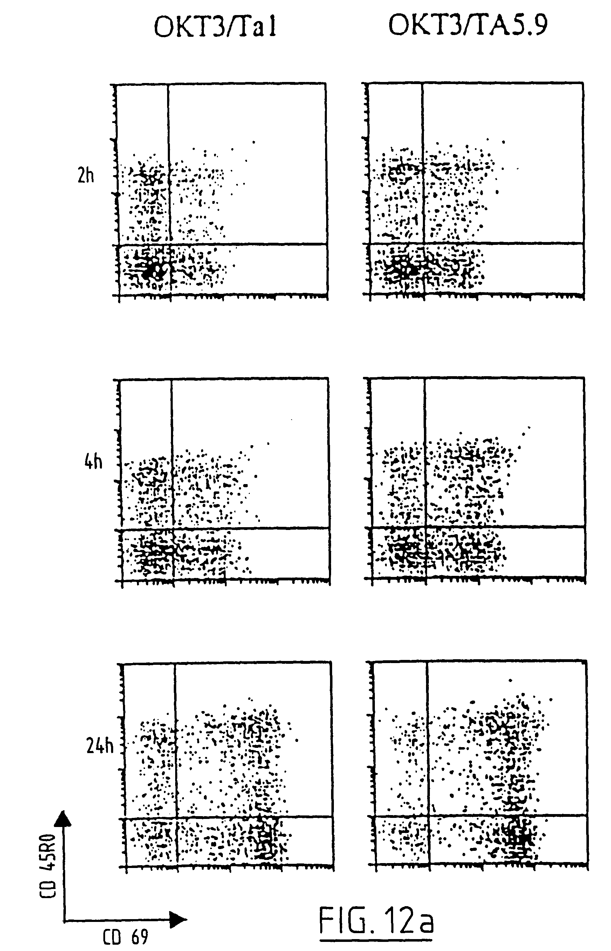

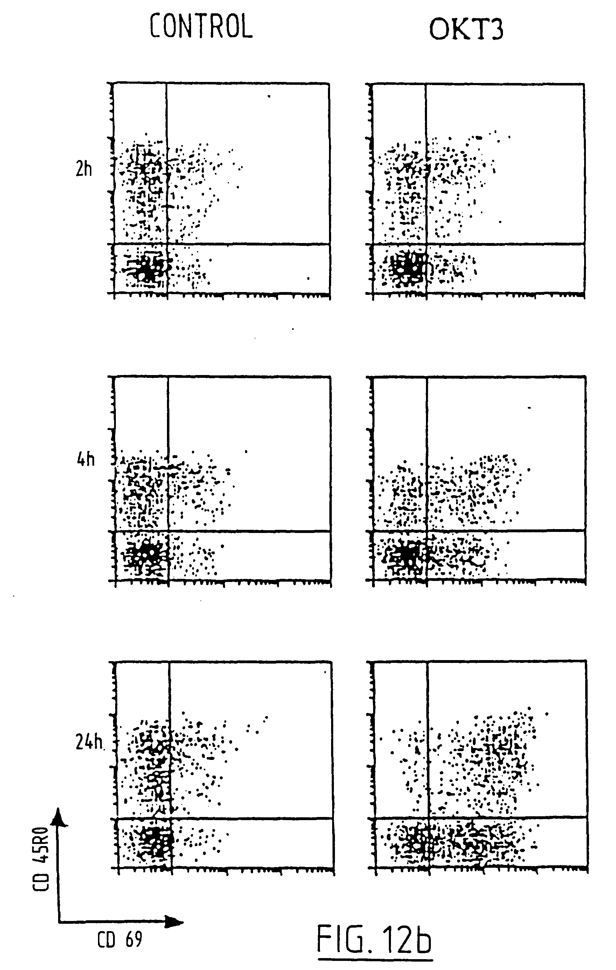

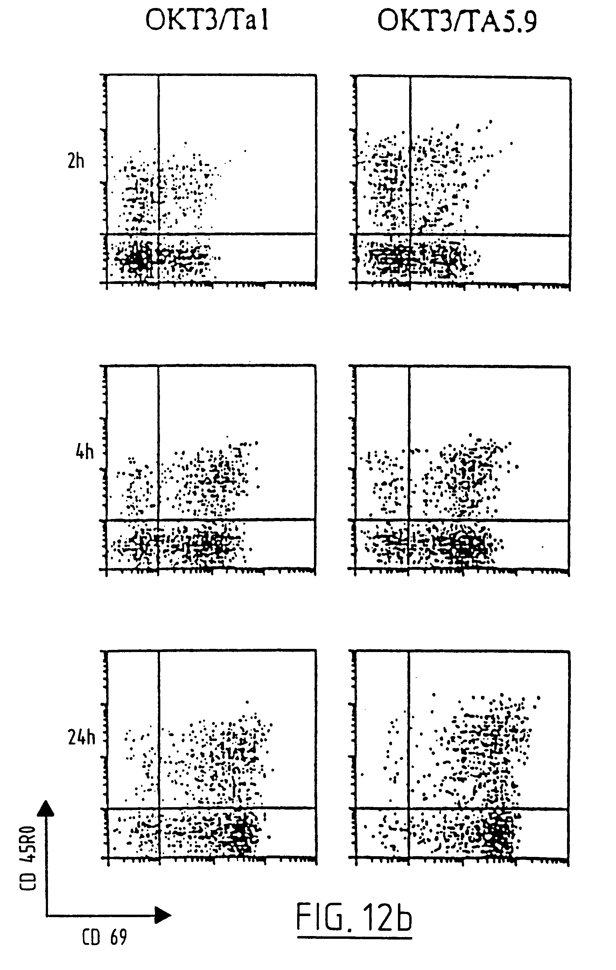

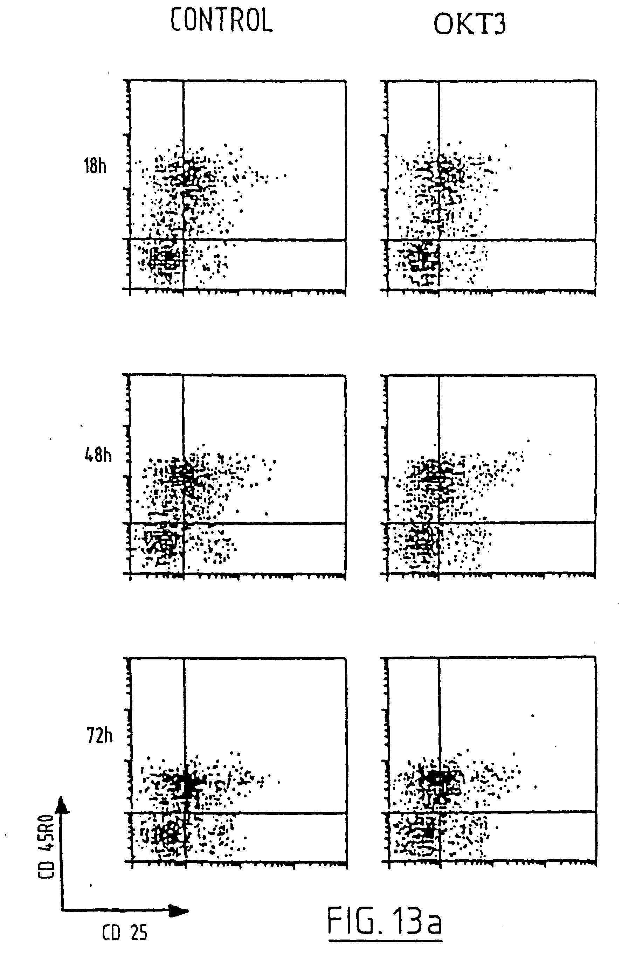

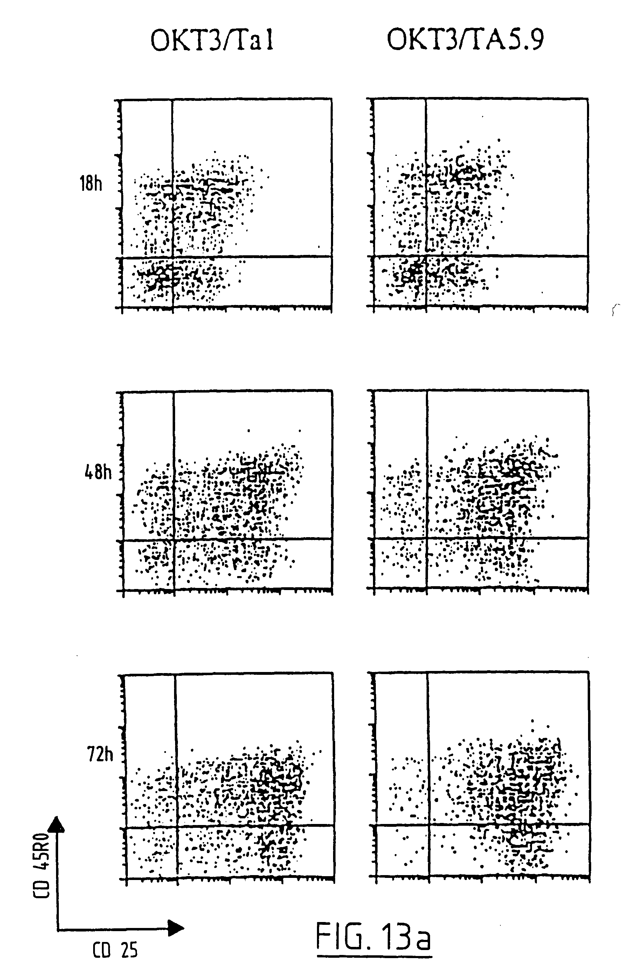

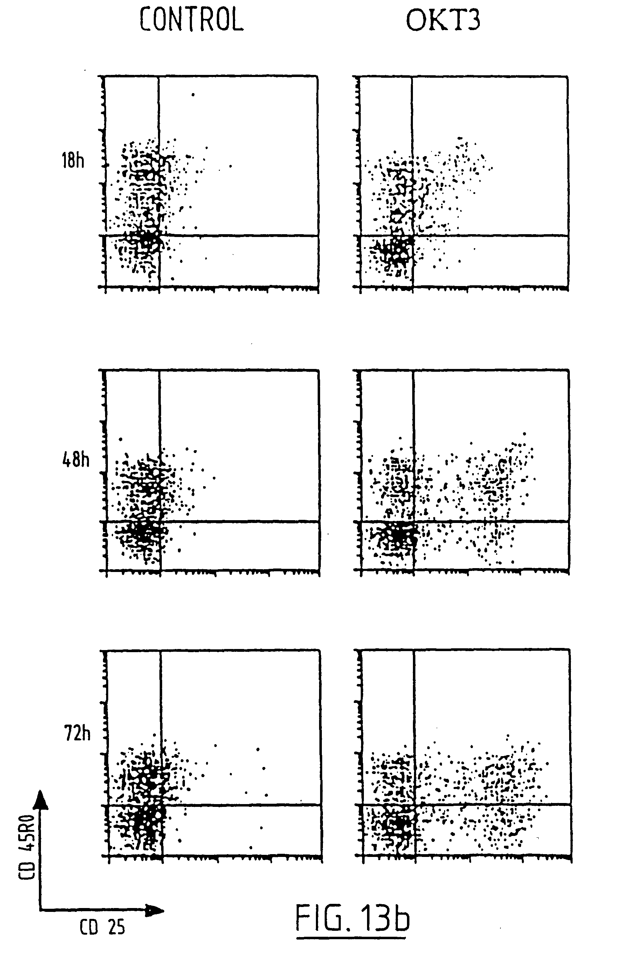

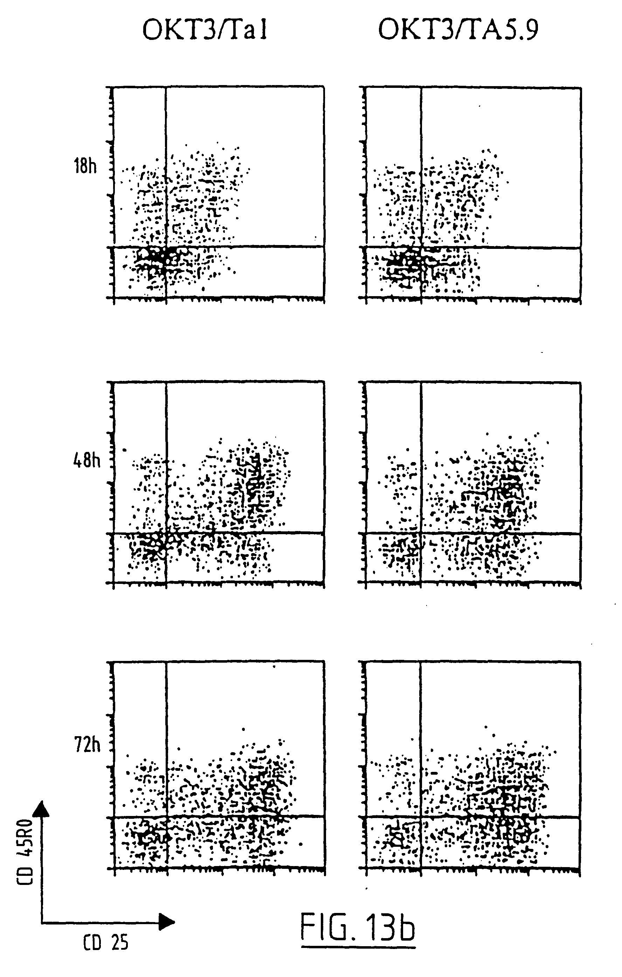

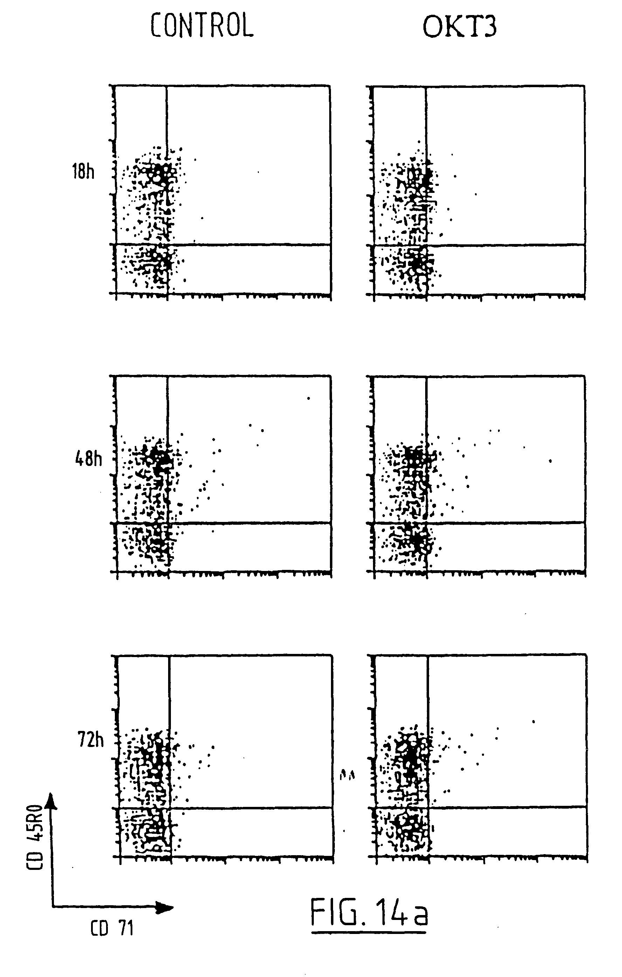

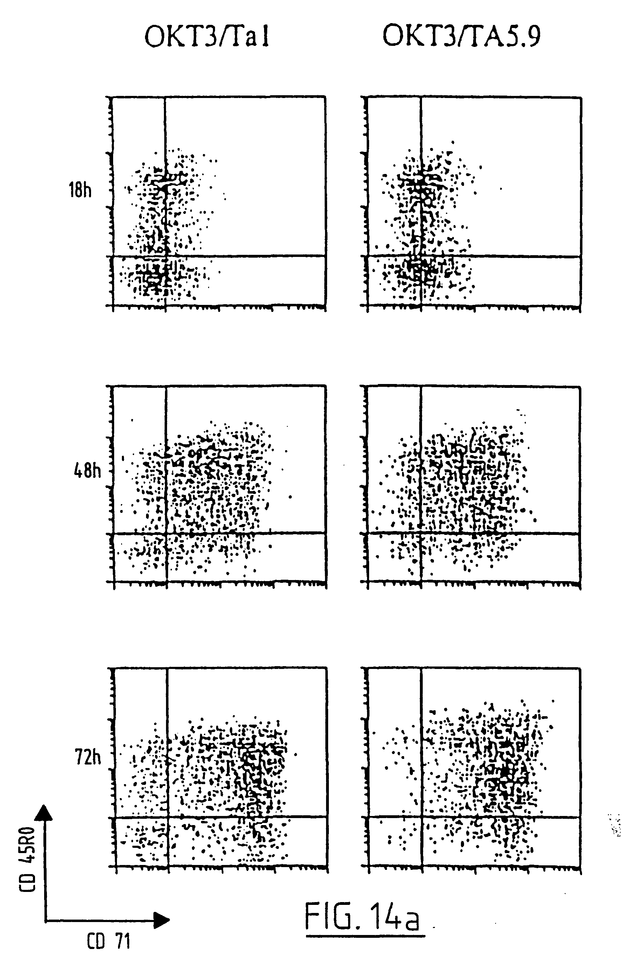

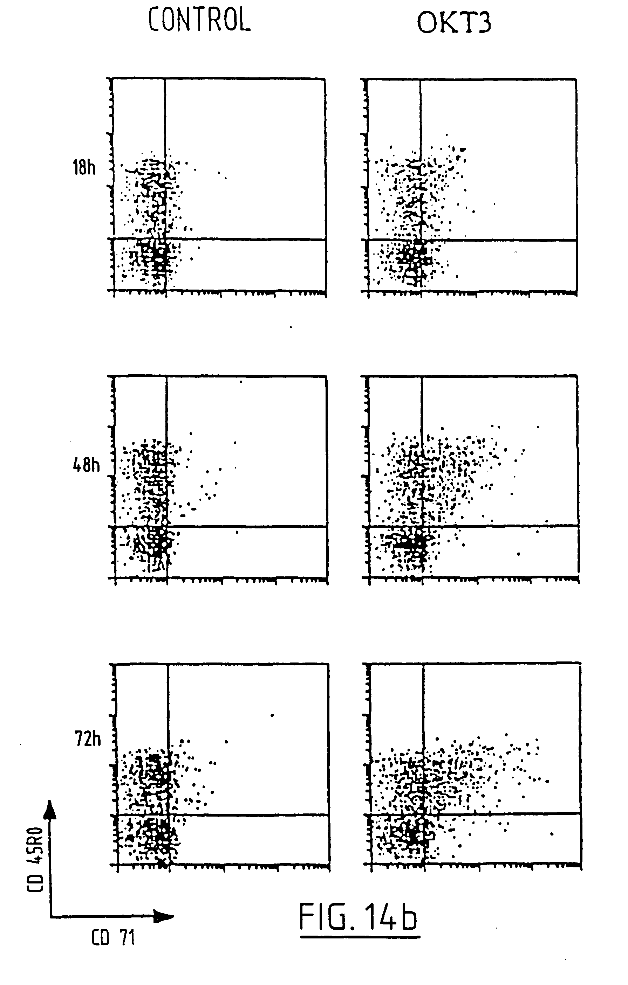

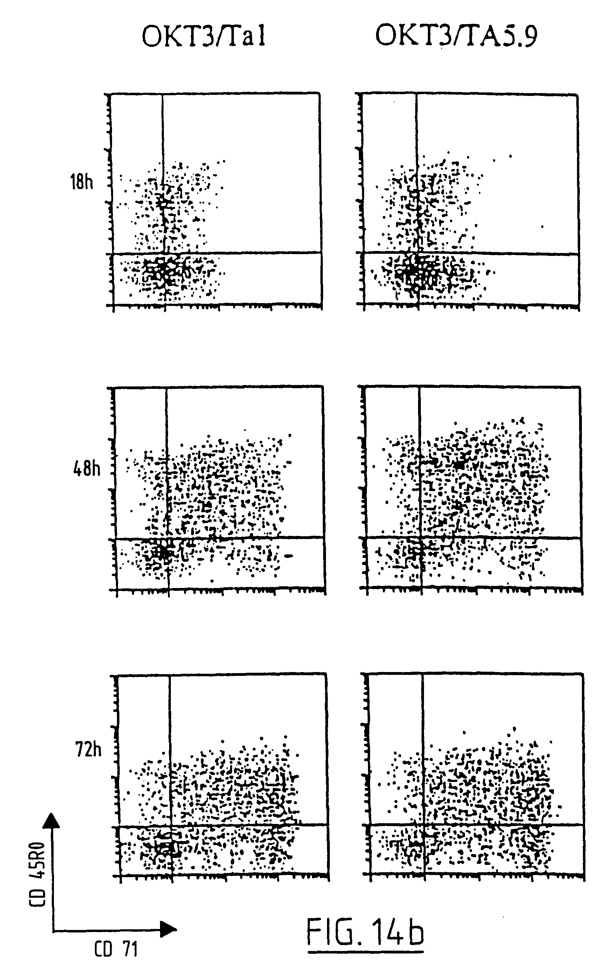

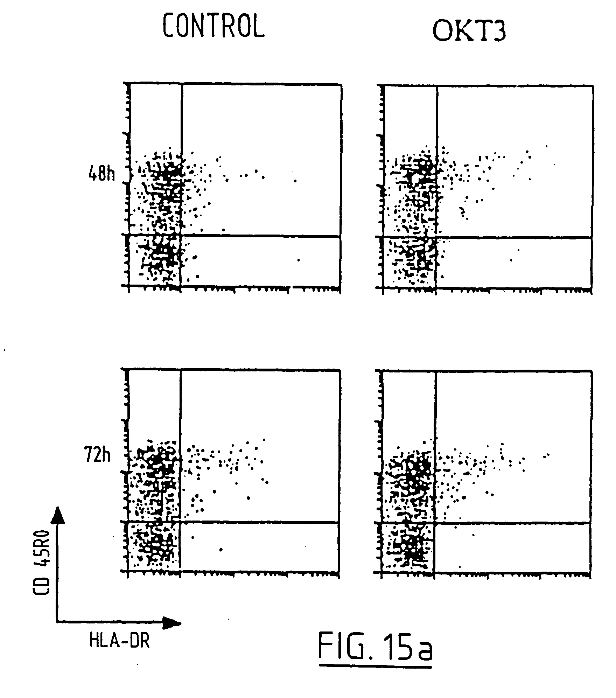

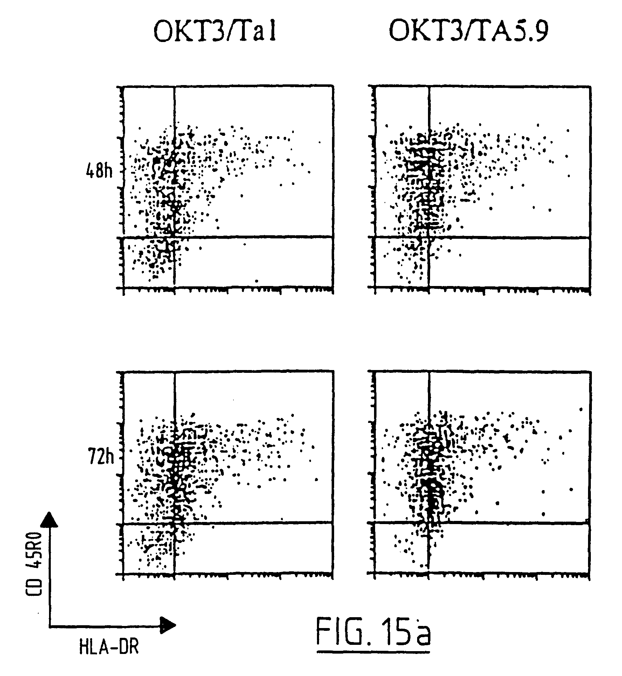

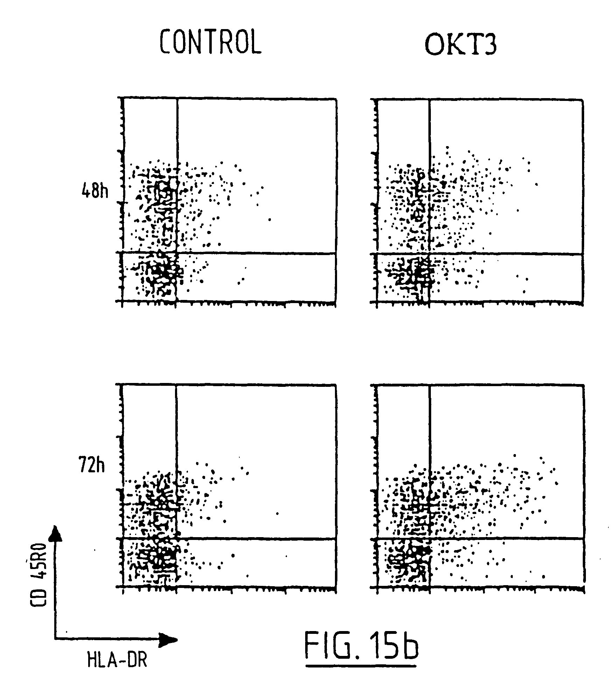

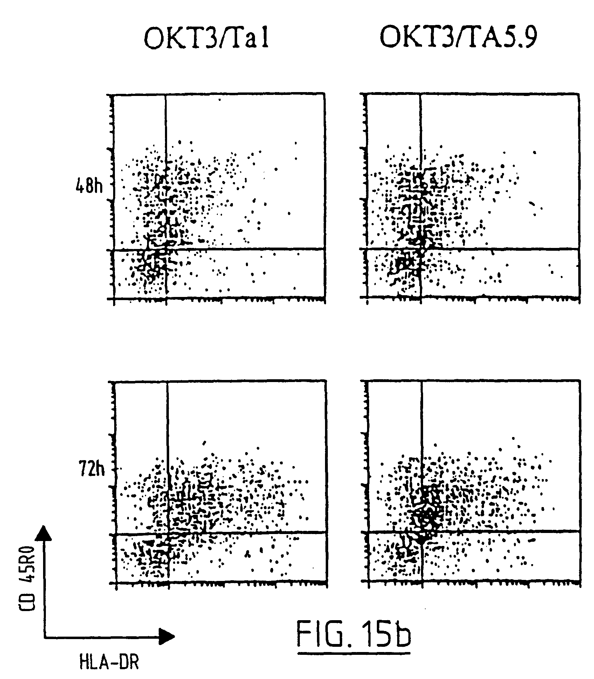

Figures 12-15 show the expression of activation markers after CD3 stimulation and CD3-CD26 co-stimulation of purified CD4+ and CD8+ T-cells. At the given times, the expression of the activation-antigen (x-axis, FITC-fluorescence) is determined on the CD45RO+ and CD45RO- cells (y-axis, PE-fluorescence) with the aid of direct immunofluorescence.

The table gives a summary of the expression of activation markers on the different T-cell subsets after CD3-CD26 co-stimulation.

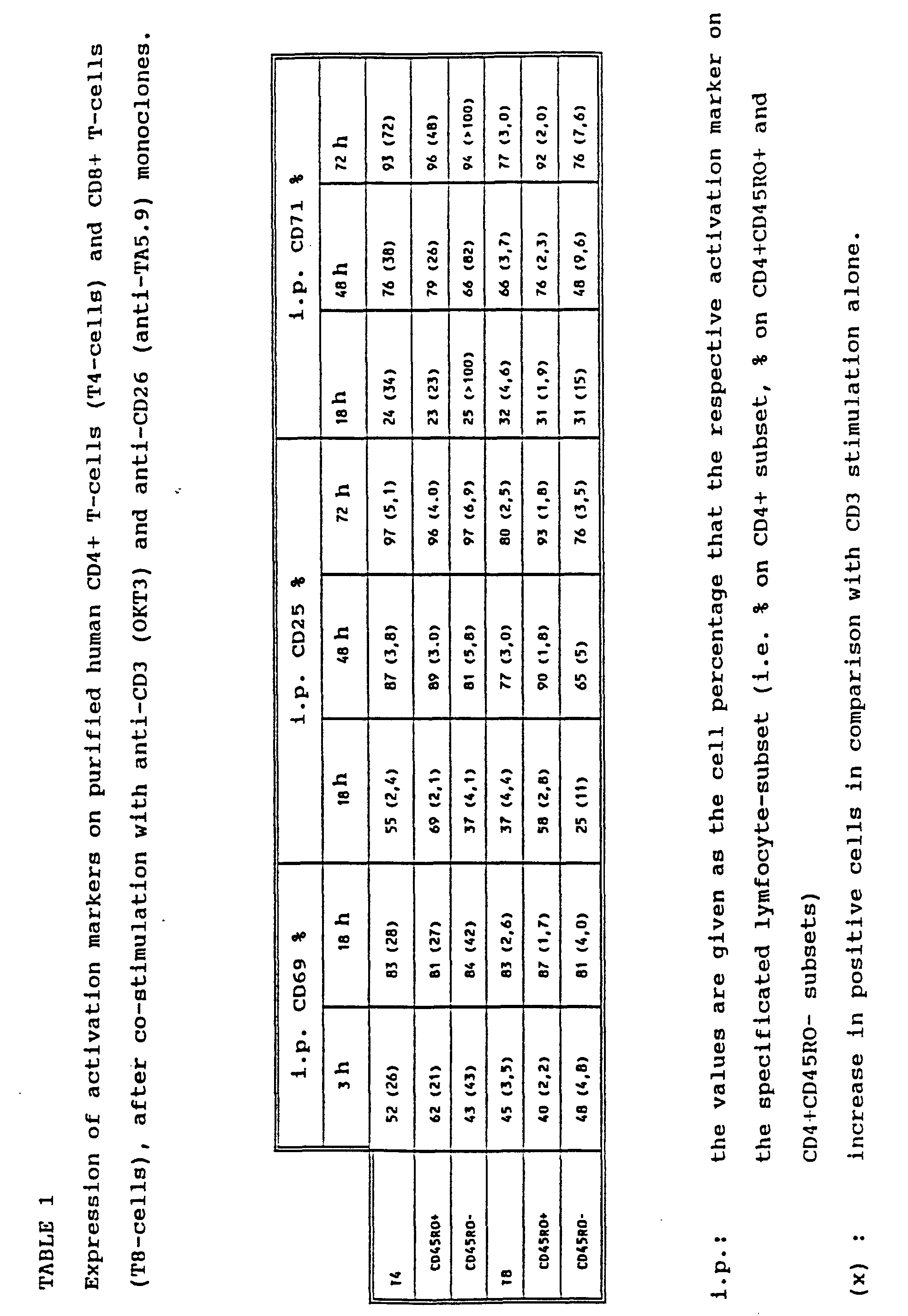

### REFERENCES

1. Giblet, E.R., Anderson, J.E., Cohen, F., Pollara, B. & Meuwissen, H.J. **Lancet** 1974. 2:1067.
2. Van Der Weyden, M. & Kelley, W.N. **J. Biol. Chem.** 1976. 251:5448.
3. Chechik, B.E., Schrader, W.P. & Minowada, J. **J. Immunol.** 1981. 126:1003.
4. Swallow, D.M., Evans, L. & Hopkinson, D.A. **Nature** 1977. 269:261.
5. Hirschhorn, R. **J. Clin. Invest.** 1975. 55:661.
6. Daddona, P.E. & Kelley, W.N. **Mol. Cell. Biochem.** 1980. 29:91.
7. Dinjens, W.N.M., ten Kate, J., van der Linden, E.P.M., Wijnen, J.T., Khan, P.M. & Bosman, F.T. **J. Histochem. Cytochem.** 1989.37:1869.
8. Morrison, M.E., Vijayasaradhi, S., Engelstein, D., Albino, A.P. & Houghton, A.N. **J. Exp. Med.** 1993. 177:1135.
9. Vanhoof, G., De Meester, I., van Sande, M., Scharpé, S. & Yaron, A. **Eur. J. Clin. Chem. Clin. Biochem.** 1992. 30:333.
10. Ulmer, A., Mattern, T., Feller, A.C., Heymann, E., & Flad, H-D. **Scand. J. Immunol.** 1990 31:429.
11. Bednarczyk, J.L., Carroll, S.M., Marin, C. & McIntyre, B.W. J. Cell. Biochem. 1991. 46:206.
12. Dang, N.H., Torimoto, Y., Deusch, K., Schlossman, S.F. & Morimoto, C. **J. Immunol.** 1990. 144:4092.
13. Schön, E., Sigbert, J., Kiessig, S.T., Demuth, H.-U., Neubert, K., Barth, A., Von Baehr, R. & Ansorge, S. **Eur. J. Immunol.** 1987. 17:1821.
14. Flentke, G.R., Munoz, E., Huber, B.T., Plaut, A.G., Kettner, C.A. & Bachovchin, W.W. **Proc. Nat. Acad. Sci. USA** 1991. 88:1556.
15. Tanaka, T., Kameoka, J., Yaron, A., Schlossman, S.F. & Morimoto C. **Proc. Natl. Acad. Sci. USA.** 1991. 33:737
16. Mattern, T., Scholz, W., Feller, A.C., Flad, H.D. & Ulmer, A.J. **Scand. J. Immunol.** 1991. 33:737.
17. Hendriks, D., De Meester, I., Umiel, T., Vanhoof, G., van Sande, M., Scharpé, S. & Yaron, A. **Clin. Chim. Acta.** 1991. 196:87.
18. Boyum, A. Isolation of mononuclear cells & granulocytes from human blood. Scand. **J. Clin. Lab. Invest.** 1968. 21:77.
19. Scharpé, S., De Meester, I., Vanhoof, G., Hendriks, D., van Sande, M., Van Camp, K. & Yaron, A. **Clin. Chem.** 1988. 34:2299.
20. De Meester, I., Scharpé, S., Vanham, G., Bosmans, E., Heyligen, H., Vanhoof, G. & Corte, G. **Immunobiol.** 1993. 188:145.
21. Andy, R.J., Finstadt, C.L., Old, L.J., Lioyd, K.O. & Kornfeld, R. **J. Biol. Chem.** 1984. 259:12844.
22. Andy, R.J. & Kornfeld, R. **J. Biol. Chem.** 1984. 259:-9832.
23. Dinjens, W.N.M., van der Boon, J., ten Kate, J., Zeijlemaker, W.P., De Bruijn, C.H.M.M., Bosman, F.T. & Khan, P.M. **Adv. Exp. Med. Biol.** 1986. 95B:407.
24. Subramanyam, M., Gutheil, W.C., Bachovchin, W.W. & Huber, B.T. **J. Immunol.** 1993. 150:2544.
25. Van Ham et al.

## Claims

1. Use of monoclonal antibody Ta 5.9 and functional derivatives thereof having at least one binding specificity for the adenosine deaminase (ADA) binding site of CD26 for recognition of the ADA binding site.

2. Use according to the claims 1, wherein the functional derivative is a F_{ab'}-, F_{ab}-, F_{(ab)2}-, a scFᵥ or Fᵥ-fragment of a monoclonal antibody having at least one binding specificity for the adenosine deaminase binding-site of CD26.

3. Use according to claims 1, wherein the functional derivative is a chimeric antibody or fragment thereof based on a monoclonal antibody having at least one binding specificity for the adenosine deaminase binding-site of CD26.

4. Use according to claims 1, wherein functional derivatives is a bispecific antibody or fragment thereof based on a monoclonal antibody having at least one binding specificity for the adenosine deaminase binding-site of CD26.

5. Use according to claims 1 and 2 wherein the monoclonal antibody Ta 5.9 is produced by the hybridoma cell line anti-CD26-(Ta5.9-CC1-4C8 having the deposit accession number ECACC 93110419.

6. Use of a conjugate between monoclonal antibody Ta 5.9 and functional derivatives thereof having at least one binding specificity for the adenosine deaminase (ADA) binding site of CD 26 and a secondary molecule for recognition of the ADA binding site.

7. Use according to claim 6 wherein the secondary molecule is selected from the group consisting of toxins, enzymes, medicines, radioactive markers, fluorescent markers, magnetic resonance markers, proteases, synthetic and natural polymers.

## Patentansprüche

1. Verwendung des monoklonalen Antikörpers TA5.9 und funktioneller Derivate davon mit mindestens einer Bindungsspezifität für die Adenosindeaminase (ADA)-Bindungsstelle von CD26 zur Erkennung der ADA-Bindungsstelle.

2. Verwendung nach Anspruch 1, wobei das funktionelle Derivat ein F_{ab'}-, F_{ab}-, F_{(ab)2}-, ein scFᵥ- oder Fᵥ-Fragment eines monoklonalen Antikörpers mit mindestens einer Bindungsspezifität für die Adenoslndeaminase-Bindungsstelle von CD26 ist.

3. Verwendung nach Anspruch 1, wobei das funktionelle Derivat ein chimärer Antikörper oder ein Fragment davon ist, der/das auf einem monoklonalen Antikörper mit mindestens einer Bindungsspezifität für die Adenosindeaminase-Bindungsstelle von CD26 basiert.

4. Verwendung nach Anspruch 1, wobei das funktionelle Derivat ein bispezifischer Antikörper oder ein Fragment davon ist, der/das auf einem monoklonalen Antikörper mit mindestens einer Bindungsspezifität für die Adenosindeaminase-Bindungsstelle von CD26 basiert.

5. Verwendung nach Anspruch 1 oder 2, wobei der monoklonale Antikörper TA5.9 von der Hybridom-Zellinie Anti-CD26-(Ta5.9-CC1-4C8) mit der Hinterlegungsnummer ECACC 93110419 produziert wird.

6. Verwendung eines Konjugats zwischen monoklonalem Antikörper TA5.9 und funktionellen Derivaten davon mit mindestens einer Bindungsspezifität für die Adenosindeaminase (ADA)-Bindungsstelle von CD26 und einem sekundären Molekül zur Erkennung der ADA-Bindungsstelle.

7. Verwendung nach Anspruch 6, wobei das sekundäre Molekül aus der Gruppe bestehend aus Toxinen, Enzymen, Medikamenten, radioaktiven Markern, Fluoreszenzmarkern, Magnetresonanzmarkern, Proteasen, synthetischen und natürlichen Polymeren ausgewählt ist.

## Revendications

1. Utilisation d'anticorps monoclonal Ta5.9 et de ses dérivés fonctionnels ayant au moins une spécificité de fixation pour le site de fixation à l'adénosine désaminase (ADA) de CD26 pour une reconnaissance du site de fixation à l'ADA.

2. Utilisation selon la revendication 1, dans laquelle le dérivé fonctionnel est un fragment F_{ab'}-, F_{ab}-, F_{(ab)2}-, scFᵥ ou Fᵥ d'un anticorps monoclonal ayant au moins une spécificité de fixation pour le site de fixation à l'adénosine désaminase de CD26.

3. Utilisation selon la revendication 1, dans laquelle le dérivé fonctionnel est un anticorps chimérique ou un fragment de celui-ci basé sur un anticorps monoclonal ayant au moins une spécificité de fixation pour le site de fixation à l'adénosine désaminase de CD26.

4. Utilisation selon la revendication 1, dans laquelle le dérivé fonctionnel est un anticorps bispécifique ou un fragment de celui-ci basé sur un anticorps monoclonal ayant au moins une spécificité de fixation pour le site de fixation à l'adénosine désaminase de CD26.

5. Utilisation selon les revendications 1 et 2, dans laquelle l'anticorps monoclonal Ta5.9 est produit par la lignée de cellules hybridomes anti-CD26-(Ta5.9-CC1-4C8) ayant le numéro d'accès de dépôt ECACC 93110419.

6. Utilisation d'un conjugué entre un anticorps monoclonal Ta5.9 et ses dérivés fonctionnels ayant au moins une spécificité de fixation pour le site de fixation à l'adénosine désaminase (ADA) de CD26 et d'une molécule secondaire pour une reconnaissance du site de fixation à l'ADA.

7. Utilisation selon la revendication 6, dans laquelle la molécule secondaire est choisie dans le groupe constitué par les toxines, les enzymes, les médicaments, les marqueurs radioactifs, les marqueurs fluorescents, les marqueurs pour résonance magnétique, les protéases, et les polymères synthétiques et naturels.
